# EUROPEAN PATENT SPECIFICATION

(11) **EP 1 746 097 B1**
(45) Date of publication and mention of the grant of the patent: **13.01.2010**
(21) Application number: 05291558.4
(22) Date of filing: 20.07.2005
(51) Int. Cl.: C07D 471/14, C07D 471/04, A61K 31/4162

(54) **1,4-dihydropyridine-fused heterocycles, process for preparing the same, use and compositions containing them**
1,4-Dihydropyridine-kondensierte Heterocyclen Verfahren zu deren Herstellung sowie Verwendung und Zusammensetzungen dergleichen
Héterocyles à fusion1,4-dihydropyridineprocédé de préparation, utilisation et compositions de celles-ci

(43) Date of publication of application: 24.01.2007
(73) Proprietor: Aventis Pharma S.A., 92160 Antony (FR)
(72) Inventor: Mauger, Jacques, 85704 Tucson (US); Nair, Anil, Oro Valley Tucson 85737 (US); Ma, Nina, 85737 Tucson (US); Bjergarde, Kirsten, Oro Valley Tucson 85737 (US); Filoche-Romme, Bruno, 94000 Creteil (FR); Angouillant-Boniface, Odile, 75014 Paris (FR); Mignani, Serge, 92290 Chatenay-Malabry (FR)
(74) Representative: Raboin, Jean-Christophe

(56) References cited:
- WO-A-99/30710
- WO-A-02/094835
- WO-A-2004/005323
- WO-A-2005/016245
- DATABASE CHEMCATS CHEMICAL ABSTRACTS SERVICE, COLUMBUS OHIO, US; XP002362347
- DATABASE CHEMCATS CHEMICAL ABSTRACTS SERVICE; COLUMBUS, OHIO, US; XP002362348
- DATABASE CHEMCATS CHEMICAL ABSTRACTS SERVICE, COLUMBUS, OHIO, US; XP002362349
- DATABASE CHEMCATS CHEMICAL ABSTRACTS SERVICE, COLUMBUS, OHIO, US; XP002362350
- DATABASE CHEMCATS CHEMICAL ABSTRACTS SERVICE, COLUMBUS OHIO, US; XP002362351
- DATABASE CHEMCATS CHEMICAL ABSTRACTS SERVICE, COLUMBUS OHIO, US; XP002362352
- DATABASE CHEMCATS CHEMICAL ABSTRACTS SERVICE, COLUMBUS OHIO, US; XP002362353
- DATABASE CHEMCATS CHEMICAL ABSTRACTS SERVICE, COLUMBUS OHIO, US; XP002362354
- DATABASE CHEMCATS CHEMICAL ABSTRACTS SERVICE, COLUMBUS OHIO, US; XP002362355
- DATABASE CHEMCATS CHEMICAL ABSTRACTS SERVICE, COLUMBUS OHIO, US; XP002362356
- DATABASE CHEMCATS CHEMICAL ABSTRACTS SERVICE, COLUMBUS OHIO, US; XP002362357
- DATABASE CHEMCATS CHEMICAL ABSTRACTS SERVICE, COLUMBUS OHIO, US; XP002362358
- DATABASE CHEMCATS CHEMICAL ABSTRACTS SERVICE, COLUMBUS OHIO, US; XP002362359
- DATABASE CHEMCATS CHEMICAL ABSTRACTS SERVICE, COLUMBUS OHIO, US; XP002362360
- DATABASE CHEMCATS CHEMICAL ABSTRACTS SERVICE, COLUMBUS OHIO, US; XP002362361
- DATABASE CHEMCATS CHEMICAL ABSTRACTS SERVICE, COLUMBUS OHIO, US; XP002362362
- DATABASE CHEMCATS CHEMICAL ABSTRACTS SERVICE, COLUMBUS OHIO, US; XP002362363
- DATABASE CHEMCATS CHEMICAL ABSTRACTS SERVICE, COLUMBUS OHIO, US; XP002362364
- DATABASE CHEMCATS CHEMICAL ABSTRACTS SERVICE, COLUMBUS OHIO, US; XP002362365
- DATABASE CHEMCATS CHEMICAL ABSTRACTS SERVICE, COLUMBUS OHIO, US; XP002362366
- DATABASE CHEMCATS CHEMICAL ABSTRACTS SERVICE, COLUMBUS OHIO, US; XP002362367
- IRENE DRIZIN ETAL.: "Structure-Activity Studies for a Novel Series of Tricyclic Dihydropyrimidines as K-ATP Channel Operators" BIORGANIC AND MEDICINAL CHEMISTRY LETTERS, vol. 12, 2002, pages 1481-1484, XP002362324

## Description

The present invention relates in particular to novel chemical compounds, particularly novel substituted dihydropyridine-fused heterocycles, to the compositions containing them and to their use as medicinal products. More particularly, the invention relates to specific partially saturated pyrrole or pyrazole fused naphtyridines or quinolines, exhibiting anticancer activity via modulation of the activity of proteins, in particular of kinases.
To date, most of the commercial compounds used in chemotherapy are cytotoxic agents, which pose considerable problems of side effects and of tolerance in patients. These effects may be limited in so far as the medicinal products used act selectively on cancer cells, with exclusion of healthy cells. One of the solutions for limiting the adverse effects of chemotherapy may therefore consist in using medicinal products which act on metabolic pathways or elements constituting these pathways, expressed mainly in cancer cells, and which would be expressed very little or not at all in healthy cells.
Protein kinases are a family of enzymes, which catalyze the phosphorylation of hydroxyl groups of specific protein residues such as tyrosine, serine or threonine residues. Such phosphorylations can widely modify the function of proteins; thus, protein kinases play an important role in regulating a large variety of cell processes, including in particular metabolism, cell proliferation, cell differentiation, cell migration or cell survival. Among the various cellular functions in which the activity of a protein kinase is involved, certain processes represent attractive targets for treating cancer-related diseases and also other diseases.

Thus, one of the objects of the present invention is to provide compositions having anticancer activity, acting in particular with respect to kinases. Among the kinases for which modulation of the activity is sought, Aurora A and B are preferred. The use of Aurora kinase inhibitors as anticancer agents has recently been reviewed in "aurora kinase inhibitors as anticancer agents, N. Keen and S. Taylor, 2004, Nature Reviews, 4, 927-936.

Many proteins involved in chromosome segregation and spindle assembly have been identified in yeast and drosophila. Disorganization of these proteins leads to non-segregation of chromosomes and to monopolar or disorganized spindles. Among these proteins, some kinases, including Aurora and Ipl1, which originate respectively from drosophila and *S. cerevisiae,* are necessary for chromosome segregation and separation of the centrosome. A human analogue of yeast Ipl1 has recently been cloned and characterized by various laboratories. This kinase, called Aurora2, Aurora A, STK15 or BTAK, belongs to the serine/threonine kinase family. Bischoff et al. have shown that Aurora2 is oncogenic and is amplified in human colorectal cancers (EMBO J, 1998, 17, 3052-3065). Examples of this have also been shown in cancers involving epithelial tumours, such as breast cancer.

Among the other kinases on which the products of the invention may act, mention may be made of FAK, KDR, Src, Tie2 and cyclin-dependent kinases, such as CDK1, CDK2, CDK4, CDK5, the latter being of particular interest in treatment of central nervous system related diseases.

It is worth to mention that one of the advantages of the instant invention is to provide with quite selective compounds. Indeed, these compounds mostly avoid inhibiting kinases involved in cellular transcription, which may result in severe side effects and/or higher toxicity towards quiescent cells. As a result, the compounds according to the invention mostly avoid inhibiting CDK7 and/or CDK9 kinases, or at least the inhibition ratio is in favour of an Aurora kinase.

The following corresponds to Applicant's believed closest prior art search for compounds of formulas (I) and (II) according to the invention:
Drizin, Irene; Holladay, Mark W.; Yi, Lin; Zhang, Henry Q.; Gopalakrishnan, Sujatha; Gopalakrishnan, Murali; Whiteaker, Kristi L.; Buckner, Steven A.; Sullivan, James P.; Carroll, William A. Structure-Activity studies for a novel series of tricyclic dihydropyrimidines as KATP channel openers (KCOs). Bioorganic & Medicinal Chemistry Letters (2002), 12(11), 1481-1484.
Drizin, Irene; Altenbach, Robert J.; Carroll, William A. **Preparation of tricyclic dihydropyrazolone and tricyclic dihydroisoxazolone as potassium channel openers.** U.S. Pat. Appl. Publ. 2002007059 A1 WO 2001066544 A2.
**New substituted benzimidazole derivatives are C-JUN N-terminal kinase inhibitors useful in the prevention and/or treatment of e.g. Inflammatory diseases, autoimmune diseases, destructive bone disorders and neurodegenerative diseases.** WO 200499190 A1.
Gross, Rene; Lajoix, Anne-Dominique; Ribes, Gerard. **Novel methods for screening Inhibitors of the Interaction of rat neuronal nitric oxide synthase and its cognate Inhibitor**. WO 2002083936 A2.
Kada, Rudolf; Knoppova, Viera; Kovac, Jaroslav; Cepec, Pavel. Reaction of 2-cyano-3-(4,5-dibromo-2-furyl)-2-propenenitrile and methyl 2-cyano-3-(4,5-dibromo-2-furyl)-2-propenenitrile and methyl 2-cyano-3-(4,5-dibromo-2-furyl)-2-propenoate with nucleophiles. Collection of Czechoslovak Chemical Communications (1984), 49(4), 984-91.
Kada, Rudolf; Kovac, Jaroslav. Furan derivatives. Part CXV. Condensation reactions of 5-arylthio- and 5-heter-oarylthio-2-furaldehydes with nitromethane. Collection of Czechoslovak Chemical Communications (1978), 43(8), 2037-40.
WO 2005/016245 discloses compounds having inhibitive activity of Pi 3-K and methods of using Pi 3-K inhibitory compounds to inhibit cancer cell growth.
WO 2005/016245 discloses compounds having inhibitive activity of PI 3-K (see below) and method of using these compounds to inhibit cancer cell growth : 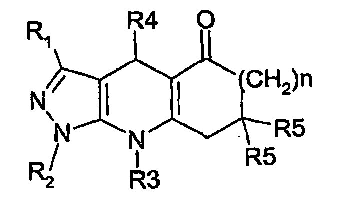 R1 and R2 can be H or an alkyl, akenyl, aryl, heteroaryl, aralkyl group, .., R3 can be H, an alkyl, alkenyl, aralkyl, groupe alkyle, alkenyle, ... and R4 can be H or an aryl, alkyl, alkenyl, cycloalkyl, aralkyl, aryl optionally substituted group. It does not describe the compounds of formula (I) where X=CR7. Moreover, it does not describe nor suggest the compounds of formula (I) where X=NH and R2 is a aryl substituted by -SR9, NHR9, CONHR9, CONHCH2R9, NHCOR9, NHCONHR9, SO2NHR9 or heteroaryl substituted.

Now, surprisingly and according to a first aspect of the invention, it has been found that products corresponding to the general formula (I) below are of particular interest for inhibiting an Aurora kinase: 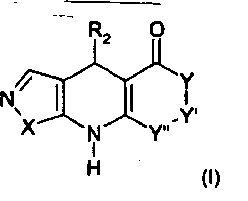 wherein, X is NH or CR₇,
- when X=NH, R₂ is :
   - aryl substituted with a substituent selected among S-R₉, NH-R₉, (C=O)-NH-R₉, (C=O)-NH-CH₂-R₉, NH-(C=O)-R₉, NH-(C=O)-NH-R₉, (SO₂)-NH-R₉
      or
   - heteroaryl substituted with a substituent selected among O-R₉, S-R₉, NH-R₉, (C=O)-NH-R₉, (C=O)-NH-CH₂-R₉, NH-(C=O)-R₉, NH-(C=O)-NH-R₉, (SO₂)-NH-R₉ ;
      when X= CR₇, R₂ is aryl or heteroaryl substituted with a substituent selected among O-R₉, S-R₉, NH-R₉, (C=O)-NH-R₉, (C=O)-NH-CH₂-R₉, NH-(C=O)-R₉, NH-(C=O)-NH-R₉, (SO₂)-NH-R₉;
      R₉ is chosen among aryl and heteroaryl optionally substituted with a substituent chosen among H, F, Cl, Br, OH, SH, CF₃, OCF₃, OCH₃, SCF₃, SCH₃, OCHF₂, OCH₂F, (C1-C6)alkyl, O-allyl, phenyl, and phenyl substituted with halogen;
- Y, Y' and Y" :
   (i) each independently represent a substituent selected among CH₂, CHR₅, CR₅R₆, C=O, O, S, NH, and NR₇; or
   (ii) together represent a substituent selected among -CH₂-O-(C=O)-. -(CH₂),- and -(CH₂)₂-chain moiety;
- R₇ represent a substituent selected among: R₉, COOR₈, COR₈, and CONHR₈;
- R₅ and R₆ each independently represent R₈;
- R₈ represents H or optionally substituted: -alkyl, -alkyl-alkylene, -alkylene, -heterocycloalkyl, - cycloalkyl, -aryl, -heteroaryl, -alkyl-heterocycloalkyl, -alkylcycloalkyl, -alkyl-aryl, or -alkylheteroaryl.

Y" is preferably CH₂.

A preferred Y is CH₂.

Y' can be particularly selected among CH₂, CHCH₃, C(CH₃)₂, CH-aryl, CH-heteroaryl, CH-(substituted aryl), CH-(substituted heteroaryl), O, S, NH, and NR7.

R2 can be particularly selected among aryl and substituted heteroaryl; wherein the substitution include a substituent chosen among halogen, OH, OR8, CH₂-ORB, SH, SR8, NH₂, and NHR8.

More particularly, R2 may be aryl or heteroaryl substituted with a substituent selected among methyl, F, Cl, Br, OH, O-R9, S-R9, NH-R9, (C=O)-NH-R9, (C=O)-NH-CH₂-R9, NH-(C=O)-R9, NH-(C=O)-NH-R9, (SO₂)-NH-R9; wherein R9 is chosen among aryl and heteroaryl, in which aryl and heteroaryl are substituted with a substituent chosen among H, F, Cl, Br, OH, SH, CF₃, OCF₃, OCH₃, SCF₃, SCH₃, OCHF₂, OCH₂F, (C1-C6)alkyl, O-allyl, phenyl, and phenyl substituted with halogen.

A preferred R2 is SR9 substituted heteroaryl, and preferred R9 is optionally substituted heteroaryl. More preferably, R9 is optionally substituted benzimidazolyl or imidazolyl.

A preferred R2 is substituted heteroaryl, wherein heteroaryl is furyl or thienyl.

X is advantageously CR7.

A preferred R7 is COOR8 or CONHR8.

A compound according to the first aspect of the invention may be in the racemic form, enriched in one enantiomer, enriched in one diastereoisomer, its tautomers, its prodrugs and its pharmaceutically acceptable salts.

According to a second aspect, the invention is about a pharmaceutical composition comprising a product according to its first aspect, in combination with a pharmaceutically acceptable excipient.

According to its third aspect, the invention is about the use of a product according to its first aspect, as an agent that inhibits the proliferation of tumour cells.

According to a fourth aspect, the invention is about the use of a product according to its first aspect, for producing a medicinal product of use in treating a pathological condition, especially a cancerous condition.

### General procedure for the synthesis of tricyclic tetrahydropyridines.

In the following schemes, R1 is H.

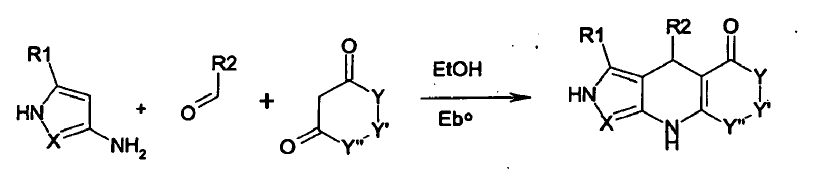

A mixture of 1.1 equivalent of pyrazole (X=N), 1 equivalent of aldehyde R2 CHO and 1 equivalent of diceto derivative is refluxed in ethanol for ½ to several hours. The solution is cooled down to room temperature. The desired compound is either isolated by filtration or the solvent is removed under vacuum. If needed, the crude is purified on silica gel or using a preparative HPLC.

When Y' is N-Boc the product is deprotected using a solution of trifluoroacetic acid in dichloromethane (50/50).

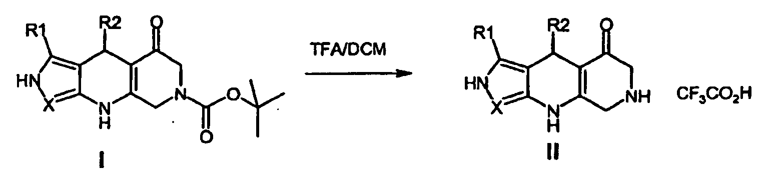

### General procedure for the preparation of N-substituted tricyclic tetrahydropyridines

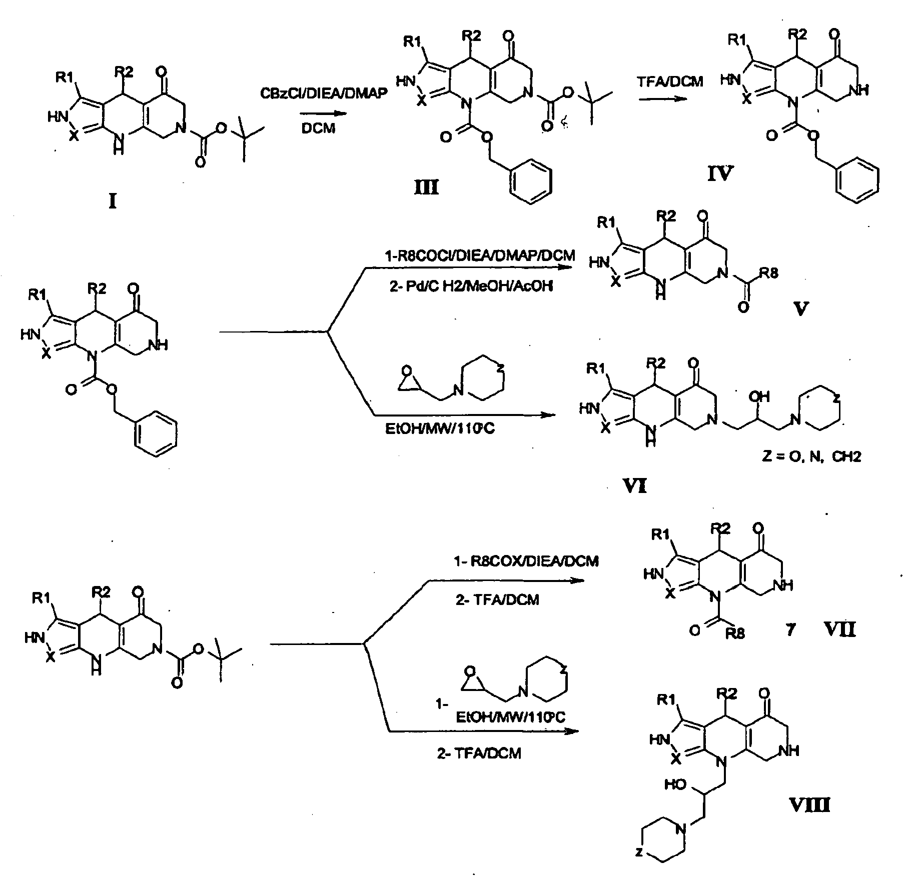

The tricyclic compound I in solution in DCM is treated by 2 equivalent of benzyloxycarbonyl chloride, 2 equivalent of diisopropylethylamine (DIEA) and catalytic amount of 4-dimethylaminopyridine (DMAP) at room temperature for 24 hours. The reaction mixture is poured into a 10% solution of potassium hydrogenosulfate and extracted with DCM. The organic phase is washed with water, dried over MgSO₄ and concentrated under vacuum. The crudes are purified on silica gel to give the Cbz protected derivative **III.**

The Boc protection is removed by treating compound **III** with a solution of TFA/DCM (50/50) at room temperature for 1 hour. After evaporation of the solvent the crude is purified on silica gel to give **IV.**

Acyl derivatives of general formula **V** are prepared in 2 steps. The compounds of formula **IV** are first acylated by various acyl chlorides in DCM using DIEA and a catalytic amount of DMAP. The reaction mixture is stirred overnight at room temperature and poured into water. The mixture is extracted with DCM. The organic phase is washed with water, dried over MgS04 and concentrated under vacuum. The compounds **V** are purified using preparative HPLC.

Alkyl derivatives of general formula **VI** are prepared from compounds **IV** using the corresponding epoxide in ethanol. The solution is either refluxed for 2 hours or irradiated with microwaves at 110°C for 10 minutes. The mixture is poured in water and extracted with DCM. The DCM solution is washed with water, dried over MgS04 and evaporated. The resulting product is hydrogenated with Pd/C under hydrogen atmosphere. After filtration on celite® and evaporation, the crude is purified using preparative LC/MS.

Acyl derivatives of general formula **VII** are prepared by direct acylation of **I** with acyl chloride as described for **III** or with acid anhydrides in DCM using DIEA for 2 hours at room temperature. The mixture is poured in 10% potassium hydrogenosulfate solution and extracted with DCM. The organic solution is washed with water, dried over MgS04 and evaporated. The crude is treated directly with a solution of TFA/DCM (50/50) for 1 hour at room temperature. The crudes are purified using preparative LC/MS to give **VII**.

Alkyl derivatives of general formula **VIII** are obtained by treating in the microwave at 150°C compound 1 with the corresponding epoxide in DMF.

The intermediate is purified using preparative HPLC. The resulting derivative is Boc-deprotected with a TFA/DCM solution (50/50). The crude is purified by preparative LC/MS.

### General procedure for the preparation of non-commercial aldehydes

### Preparation of aldehydes of general structures IX:

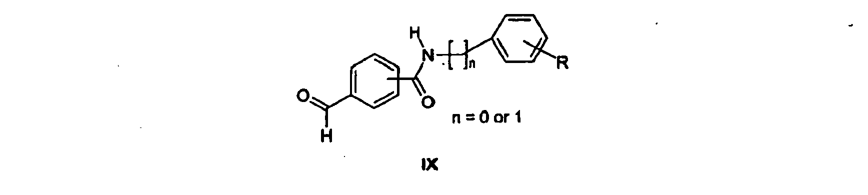

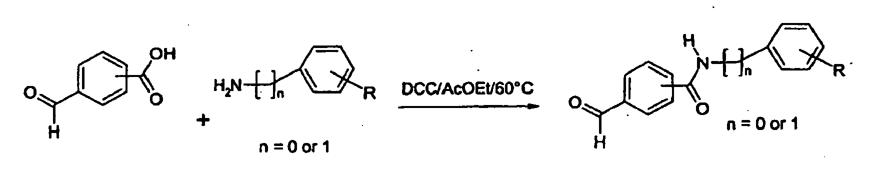

A mixture of 1 equivalent of formyl benzoic acid and 1 equivalent of aniline or benzylamine in ethyl acetate (AcOEt) is treated with dicyclohexylcarbodiimide (DCC) at 60°C for several hours. The mixture is poured in HCl 1 N. The organic phase is collected and successively washed with water, a solution of sodium bicarbonate and brine. The solution is dried over MgS04 and evaporated. The crudes are purified on silica gel when needed.

### Preparation of aldehydes of general structure X:

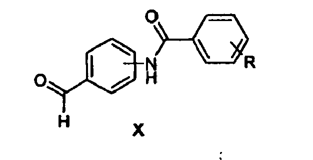

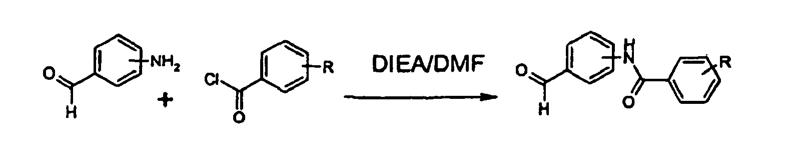

A mixture of 1 equivalent of aminobenzaldehyde and 1 equivalent of benzoyl chloride in DMF is treated with 2 equivalents of DIEA under microwave conditions at 110°C for 10 minutes. The compounds **10** generally precipitate and are collected by filtration.

### Preparation of aldehydes of general structure XI:

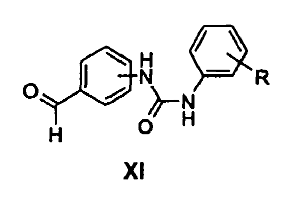

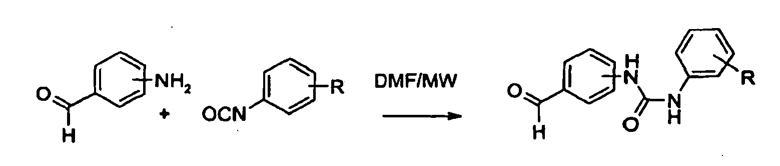

A mixture of 1 equivalent of aminobenzaldehyde and 1 equivalent of phenylisocyanate in DMF is treated under microwave conditions at 110°C for 10 minutes. The compounds **XI** generally precipitate and are collected by filtration.

### Preparation of aldehydes of general structures XII:

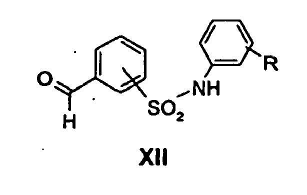

A mixture of 1 equivalent of Chlorosulfonylbenzaldehyde and 1 equivalent of aniline in DCE is treated with an exces of pyridine for several hours. The mixture is poured into 10% HCl solution and extracted with DCM. The organic phase is washed with brine, dried over MgS04 and evaporated. The crudes are purified on silica gel to give aldehydes 12.

### Preparation of aldehydes of general structure XV and XVI:

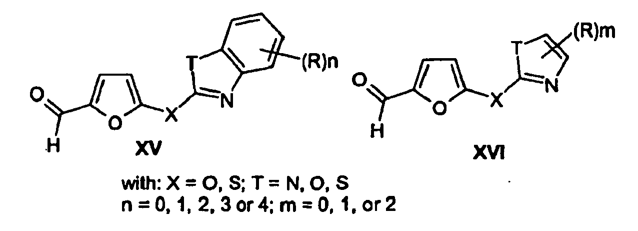

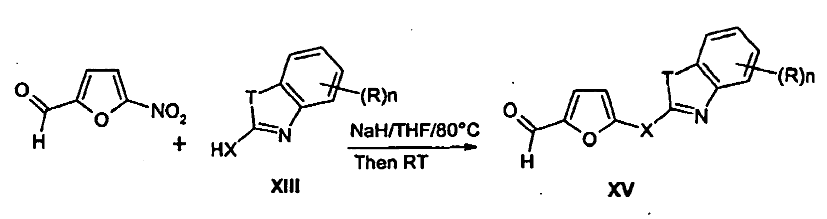

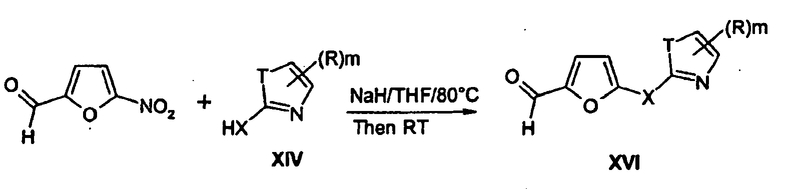

To a solution of 1 equivalent of the compound of the general formula **XIII** or **XIV** in dry THF is added 1 equivalent of NaH suspension at room temperature. The mixture is heated at 80°C for 30 minutes. The reaction mixture is cooled down to room temperature and a solution of 1 equivalent of 5-nitro-furalaldehyde in THF is added. The reaction mixture is stirred until completion of the reaction and then poured into water and extracted with ethyl acetate. The organic phase is washed with brine, dried over MgS04 and evaporated. When needed the crudes are purified on silica gel to yield compound of formula **XV** or **XVI.**

### Preparation of aldehydes of general structure XVII and XVIII:

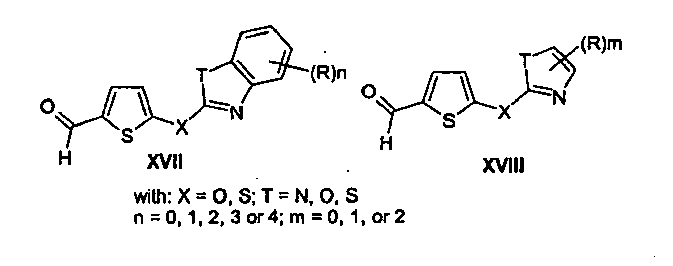

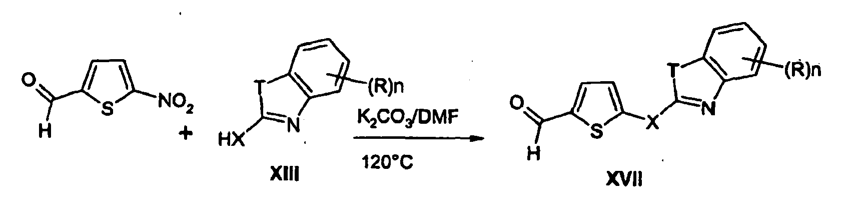

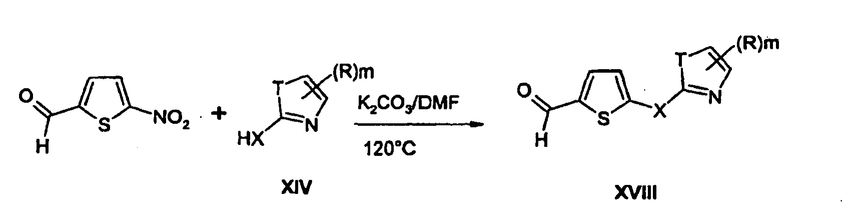

A mixture of 1 equivalent of 5-bromo-2-thiophenecarboxaldehyde, 1 equivalent of general formula **XIII** or **XIV** and 2 equivalents of potassium carbonate are heated at 120°C until completion of the reaction. Then the reaction mixture is poured in water and extracted with ethyl acetate. The organic phase is washed with brine, dries over MgS04 and evaporated. The crudes are purified on silica gel when needed to yield compounds XVII or **XVIII.**

### Experimental Part

### Example 1:4-(4-Hydroxy-3-methyl-phenyl)-1,4,6,7,8,9-hexahydro-1,2,7,9-tetraaza-benz[f]inden-5-one; compound with trifluoro-acetic acid (not part of the invention)

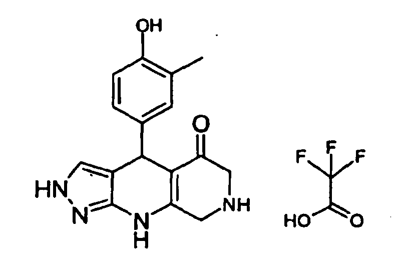

To a mixture of 213 mg of N-Boc-3, 4-diketopiperidine (1 mmole) and 83 mg aminopyrazole (1 mmole) in 5ml of ethanol is added 136.2 mg of 4-hydroxy-3-methylbenzaldehyde (1 mmole). The mixture is refluxed for 2 hours and cooled down to room temperature. The precipitate is collected by filtration and washed with ethanol to give 316 mg of pale yellow solid (yield= 80%). Retention time (RT) = 3.8 min (YMC basic S5 column; 2-85% ACN/H2O gradient over 7 min) (EIMS ([M+H]+): 397).

100 mg of the isolated compound is dissolved in 5 ml of DCM and treated with 5ml of TFA for 1 hr at room temperature. After evaporation of the solvent the crude is directly purified using preparative reverse phase HPLC. 50 mg of desired compound is isolated after lyophilisation of the fractions (yield= 50%) (YMC basic S5 column; 2-85% ACN/H2O gradient over 7 min) EIMS ([M+H]+):297. RT= 2.31 H¹NMR (D₆-DMSO) (300MHz) : 1.99 (s, 3H); 3.64 (AB, 2H); 4.09 (AB, 2H); 4.97 (s, 1 H); 6.52 (d, 1 H); 6.76 (d, 1 H); 6.80 (s, 1 H); 7.31 (s, 1 H); 10.4 (s, 1 H).

### Example 2:4-[3-(4-Chloro-phenoxy)-phenyl]-2,4,6,7,8,9-hexahydro-1,2,7,9-tetraaza-benz[f]inden-5-one; compound with trifluoro-acetic acid (not part of the invention).

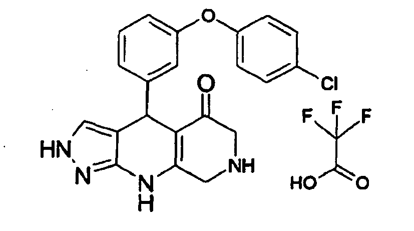

To a mixture of 107 mg of N-Boc-3, 4-diketopiperidine (0.5 mmole) and 42 mg aminopyrazole (0.5 mmole) in 2.5ml of ethanol is added 0.116 ml of 3-(4-chlorophenoxy)-benzaldehyde (0.5 mmole). The mixture is refluxed for 1/2 hour and cooled down to room temperature. The solution is concentrated under vacuum. The resulting oily residue is dissolved in 2.5 ml of DCM and treated with 2.5 ml of TFA at room temperature for 1 hour. After evaporation of the solvent the crude is directly purified using preparative reversed phase HPLC resulting 70mg of white solid after lyophilisation of the fractions (yield= 31 %). (YMC basic S5 column; 2-85% ACN/H2O gradient over 7 min) EIMS ([M+H]+): 393. RT= 3.91
H¹NMR (D₆-DMSO) (300MHz) : 3.70 (AB, 2H); 4.18 (AB, 2H); 5.17 (s, 1 H); 6.65 (d, 1 H); 6.94 (m, 4H); 7.22 (m, 1H); 7.45 (m, 3H); 9.80 (sl, 2H); 10.4 (s, 1H)

### Example 3: 4-[5-(1H-Benzimidazol-2-ylsulfanyl)-furan-2-yl]-2,4,6,7,8,9-hexahydro-pyrazolo[3,4-b]quinolin-5-one

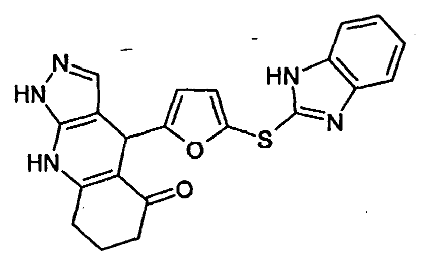

To a mixture of 225 mg of 1,3-diketopiperidine (2 mmoles) and 183 mg aminopyrazole (2.2 mmoles) in ethanol is added 490 mg of 5-(1-H-benzimidazol-2-sulfanyl)furan (2 mmoles). The mixture is refluxed for 1/2 hour and cooled down to room temperature. The precipitate is collected by filtration and washed with ethanol to give 460 mg of pale yellow solid (yield= 58%)
(YMC basic S5 column; 2-85% ACN/H2O gradient over 7 min) (EIMS ([M+H]+): 404). RT= 3.13min.
H¹NMR (D₆-DMSO) (300MHz): 1.96 (m, 2H); 2.26 (m, 2H); 2.54 (m, 2H); 4.11 (s, 1 H); 5.22 (s, 1 H); 5.97 (s, 1 H); 6.85 (s, 1 H); 7.16 (m, 2H); 7.37 (m, 1 H); 7.46 (s, 1 H); 7.55 (m, 1 H); 10.0 (s, 1 H); 12.2 (s, 1 H); 12.47 (s, 1H).

### Example 4: 4-[5-(1H-Benzimidazol-2-ylsulfanyl)-furan-2-yl]-7,7-dimethyl-2,4,6,7,8,9-hexahydro-pyrazolo[3,4-b] quinolin-5-one

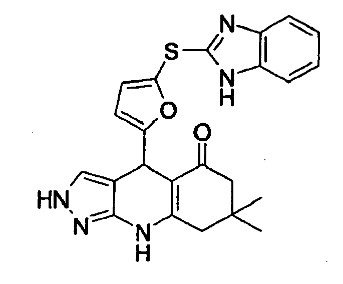

To a solution of 156 mg of dimedone (1 mmole) and 91 mg of 3-aminopyrazole (1.1 mmole) in 5 ml of ethanol is added 244mg of 5-(1 H-benzimidazol-2-ylsulfanyl)-furan-2-carbaldehyde (1 mmole). The mixture is heated at reflux for 1 hr. The solution is cooled down to room temperature and evaporated under vacuum. The crude is directly purified on silica gel using a mixture of dichloromethane/methanol 98/2 then 96/4 as eluent.

After evaporation of the fractions, 250mg of a pale yellow solid is isolated (58%). (EIMS ([M+H]+): 432). Ret. Time: 2.56 min
H¹NMR (D₆-DMSO) (300MHz): 0.81 (s, 3H); 0.87 (s, 3H); 2.00 (AB, 2H); 2.29 (AB, 2H); 5.02 (s, 1 H); 5.85 (s, 1 H); 6.72

The following example was prepared using the same procedure as for example 1:

**Table1**

| Examples | Chemistry | MW parent compound | EIMS ([M+H]⁺ | RT (min) |
|---|---|---|---|---|
| 20 | 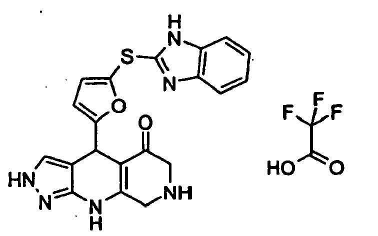 | 404.11 | 405.48 | 1.85 |
| LC/MS conditions: gradient 2 to 80 % acetonitrile in 7 min *LC/MS conditions: gradient 5 to 85 % acetonitrile in 7 min | | | | |

The following examples were prepared using the same procedure as for example 3 or 4.

**Table2**

| Examples | Chemistry | MW parent compound | EIMS ([M+H]⁺ | RT (min) |
|---|---|---|---|---|
| 28 | 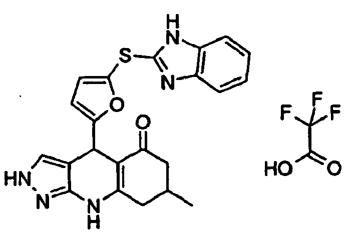 | 417.00 | 418.00 | 3.01 |
| 29 | 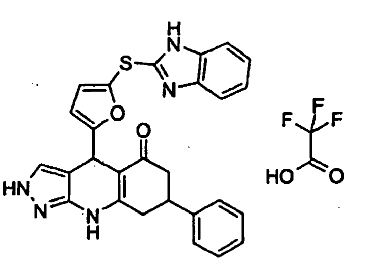 | 479.00 | 480.00 | 3.66 |
| 30 | 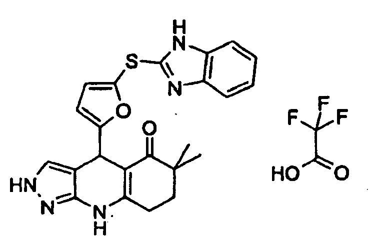 | 431.00 | 432.00 | 3.33 |
| 31 | 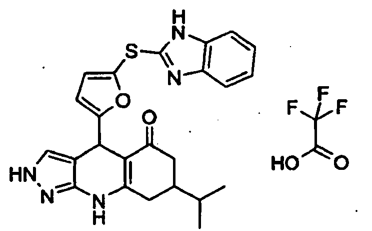 | 445.00 | 446.00 | 3.57 |
| 32 | 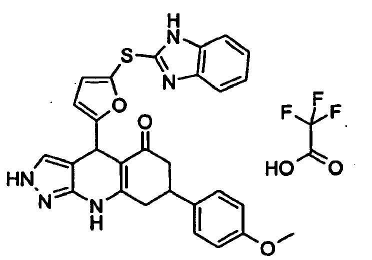 | 509.00 | 510.00 | 3.67 |
| 33 | 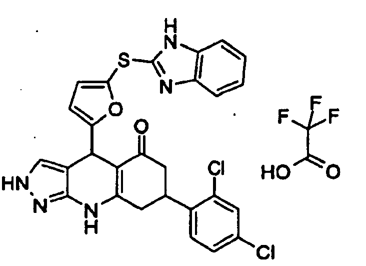 | 548.00 | 549.00 | 4.27 |
| 34 | 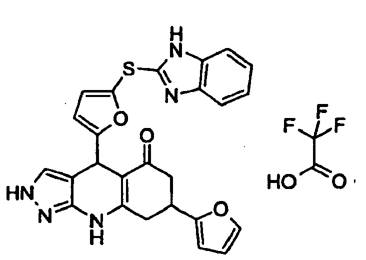 | 469.00 | 470.00 | 3.38 |
| 35 | 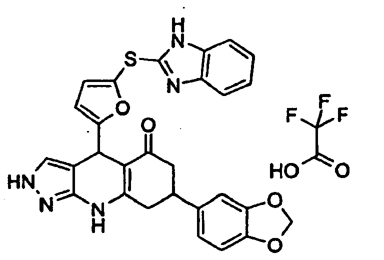 | 523.00 | 524.00 | 3.61 |
| 36 | 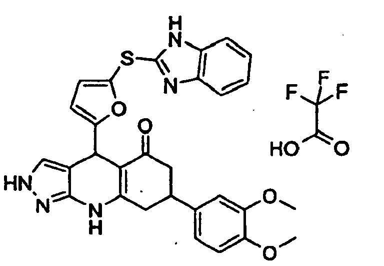 | 539.00 | 540.00 | 3.38 |
| 37 | 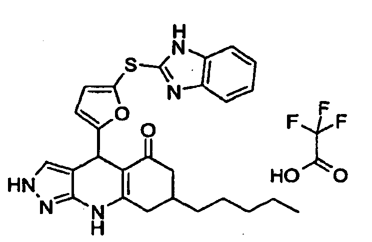 | 473.00 | 474.00 | 4.25 |
| 38 | 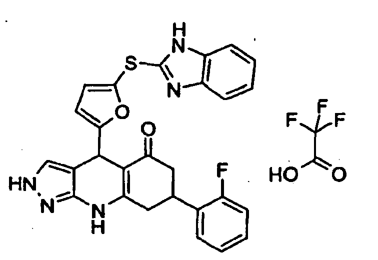 | 497.00 | 498.00 | 3.73 |
| 39 | 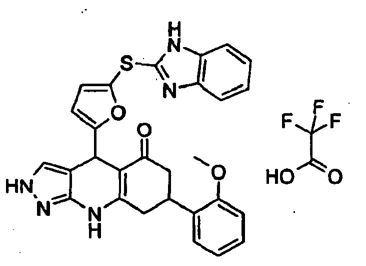 | 509.00 | 510.00 | 3.77 |
| 40 | 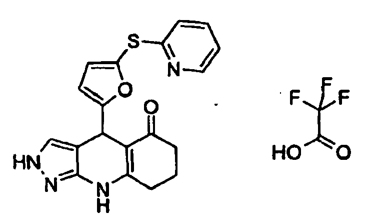 | 364.00 | 365.00 | 2.75 |
| 41 | 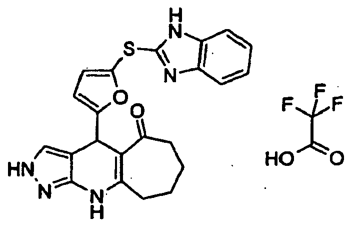 | 417.13 | 418.13 | 2.95 |
| 42 | 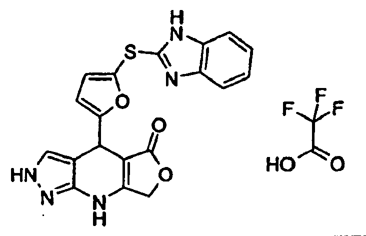 | 391.07 | 392.07 | 2.58 |

| | | | | |
|---|---|---|---|---|
| LC/MS conditions: gradient 2 to 80 % acetonitrile in 7 min *LC/MS conditions: gradient 30 to 90 % acetonitrile in 7 min | | | | |

### Example 45: 4-[5-(1H-Benzimidazol-2-ylsulfanyl)-furan-2-yl]-7-(2-hydroxy-3-piperidin-1-yl-propyl)-2,4,6,7,8,9-hexahydro-1,2,7,9-tetraazabenz[f]inden-5-one; compound with trifluoro-acetic acid

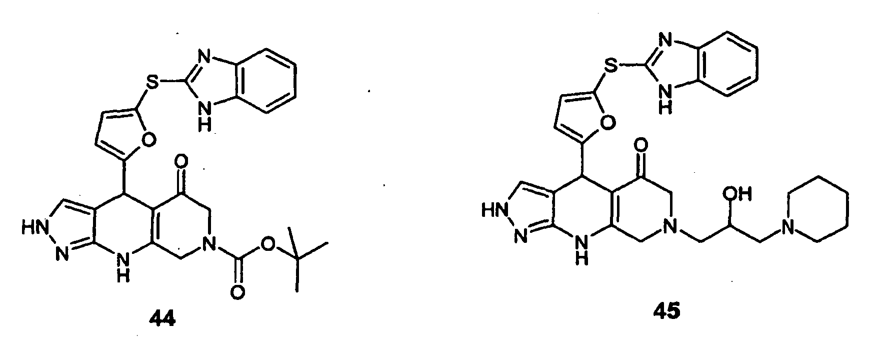

To a mixture of 443 mg of N-Boc-3, 4-diketopiperidine (2.08 mmoles) and 173 mg of 3-aminopyrazole (2.08 mmoles) in 10 ml of ethanol is added 508 mg of 5-(1H-benzimidazol-2-ylsulfanyl)-furan-2-carbaldehyde 2.08 mmoles). The mixture is refluxed for 1 hour and cooled down to room temperature. The solution is evaporated and the crude is purified on silica gel using DCM/MeOH (95/5) as eluent. 590 mg of pale yellow solid **44** is isolated (yield= 56%). (EIMS ([M+H]+): 505; Ret. Time: 3.79 min).

300 mg of the solid **44** (0.59 mmole) is dissolved in 4 ml of DCM and 0.184 ml of benzylchloroformate (1.3 mmole) is added followed by 0.385 ml of DIEA (2.36 mole) and 20 mg of DMAP (0.16 mmole). The reaction mixture is shaked overnight at room temperature then poured into 80 ml of 10% (w/v) solution of KH₂SO₄ and extracted twice with 40 ml of ethyl acetate. The combined organic phases are washed with brine, dried over MgSO₄ and evaporated. The residue is directly treated with 10 ml of a solution of TFA/DCM (50/50) for 1 hour at room temperature. The solution is concentrated under vacuum. Half of the resulting residue is dissolved in 2 ml of ethanol and directly used for the final step. The solution is treated under microwave condition with an excess of freshly prepared excess of 4-(2,3-epoxypropyl) piperidine (prepared by stirring 274 mg of epibromhydrin (2 mmoles) and 0.198 ml of piperidine (2 mmoles) in 10 ml of methanol overnight at room temperature). The mixture is microwaved 15 minutes at 150°C. The solution is evaporated and the resulting residue is purified using a preparative HPLC. 29 mg of expected compound **45** is isolated (yield=4%). ([M+H]+): 546). Ret. Time: 2.67 min.

### Example 50: 4-[3-(4-Chloro-phenoxy)-phenyl]-7-(2-hydroxy-3-morpholin-4-yl-propyl)-2,4,6,7,8,9-hexahydro-1,2,7,9-tetraazabenz[f]inden-5-one; compound with trifluoro-acetic acid (not part of the invention)

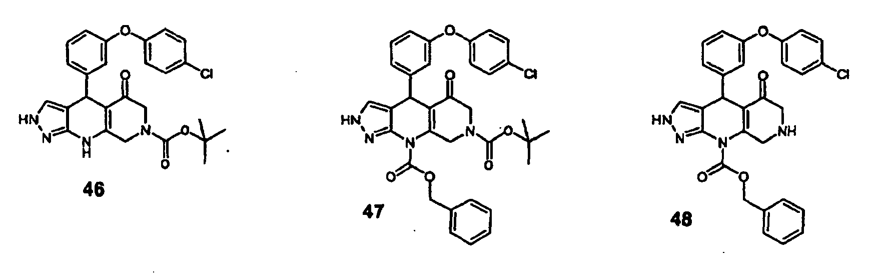

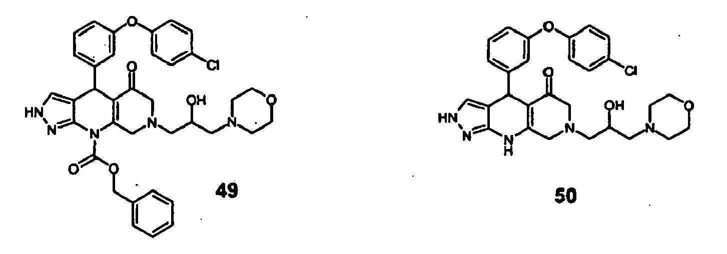

The compound **50** is prepared using the same procedure as example **45** using 3-(4-chlorophenoxy)-benzaldehyde as aldehyde. To a mixture of 1.07 g of N-Boc-3, 4-diketopiperidine (5 mmoles) and 415 mg of 3-aminopyrazole (5 mmoles) in 10 ml of ethanol is added 962 mg of 3-(4-chlorophenoxy)benzaldehyde (5 mmoles). The mixture is refluxed for 1 hour and cooled down to room temperature. The solution is evaporated and the crude is purified on silica gel using DCM/MeOH (95/5) as eluent. 1.33 g of pale yellow solid **46** is isolated (yield= 54%). (EIMS ([M+H]+): 493; Ret. Time: 3.28 min) (gradient 30 to 90 % acetonitrile in 7 min).

1.21g of the solid (2.46 mmoles) is dissolved in 20 ml of DCM and 0.184 ml of benzylchloroformate (4.92 mmoles) is added followed by 1.6 ml of DIEA (9.84 moles) and 20 mg of DMAP (0.16 mmole). The reaction mixture is shaked overnight at room temperature then poured into 80 ml of 10% (w/v) solution of KH₂SO₄ and extracted twice with 40 ml of ethyl acetate. The combined organic phases are washed with brine, dried over MgSO₄ and evaporated. The crude is purified on silica gel using a solution of DCM/MeOH 98/2 as eluent. 1.6 g of desired compound **47** is isolated (quantitative yield) (EIMS ([M+H]+): 627; Ret. Time: 4.52 min) (gradient 30 to 90 % acetonitrile in 7 min). The product **47** is treated with 20 ml of a solution of TFA/DCM (50/50) for 1 hour at room temperature. The solution is concentrated under vacuum. The resulting product is purified on silica gel using DCM/MeOH 90/10 as eluent. 0.91 g of expected compound **48** is isolated (yield=64%) (EIMS ([M+H]+): 527; Ret. Time: 2.66 min) (gradient 30 to 90 % acetonitrile in 7 min).

A solution of 53 mg of **48** (0.1 mmole) and 21 mg of epoxypropyl) morpholine (0.15 mmole) in 1 ml of ethanol is refluxed for 2 hours. After cooling the mixture is poured into 80 ml of water and extracted twice with 50 ml of DCM. The combined organic phases are washed with 50 ml of 0.5N HCl, with brine, dried over MgS04 and evaporated. The residue **49** is directly hydrogenated under hydrogen atmosphere using 0.01 mmole of palladium over carbon. The reaction is kept overnight under stirring and the reaction mixture is filtrated over celite. The filtrate is evaporated and the crude is directly purified using preparative LC/MS. 45 mg of the desired compound **50** is isolated (yield=85%). ([M+H]+): 536). Ret. Time: 3.77 min (gradient 5 to 85 % acetonitrile in 7 min).

### Example 52: 4-[5-(1H-Benzimidazol-2-ylsulfanyl)-furan-2-yl]-9-(2-hydroxy-3-morpholin-4-yl-propyl)-2,4,6,7,8,9-hexahydro-1,2,7,9-tetraazabenz[f]inden-5-one; compound with trifluoro-acetic acid (not part of the invention)

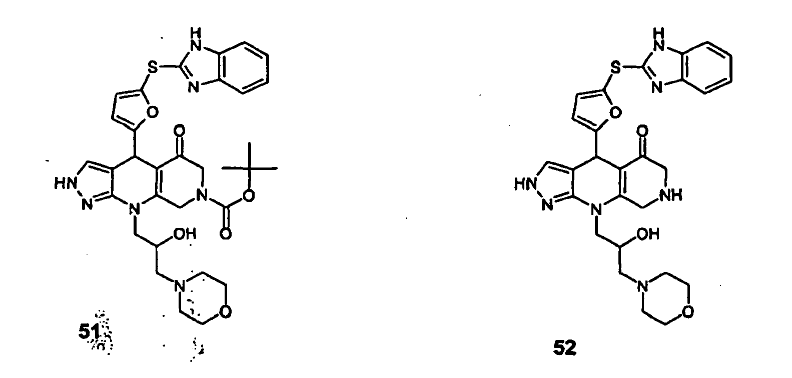

To a solution of 50 mg of compound **44** (0.1 mmole) in 1 ml of EtOH and 1 ml of DMF is added 21 mg of 4-(2,3-epoxypropyl)morpholin (0.15 mmole). The solution is heated at 150 °C in a microwave for 20 minutes. The solution is evaporated and the intermediary **51** is purified by preparative LC/MS. The compound **51** is treated with 2 ml of a TFA/DCM (50/50) solution for 1 hour at room temperature. The final product is purified using preparative LC/MS. 19 mg of compound **52** is isolated (yield= 29%). ([M+H]+): 548). Ret. Time: 2.58 min (gradient 5 to 85 % acetonitrile in 7 min).

### Example 53: 4-[3-(4-Chloro-phenoxy)-phenyl]-7-(3,5-dimethyl-isoxazole-4-carbonyl)-2,4,6,7,8,9-hexahydro-1,2,7,9-tetraaza-benz[f]inden-5-one; compound with trifluoro-acetic acid (not part of the invention)

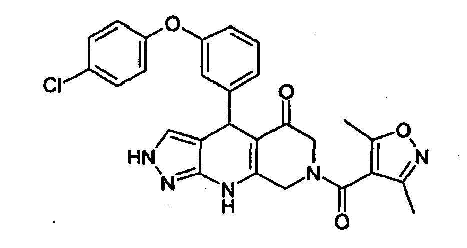

To a solution of 26.3 mg (0.05 mmole) of compound **48** in 0.5 ml of DCM is successively added 12 mg of 3,5-dimethylisoxazole-4-carbonyl (0.75 mmole), 16 ul of DIEA (0.1 mmole) and 5 mg of DMAP (0.04 mmole). The reaction is stirred overnight and the reaction mixture is poured into 20 ml of water and extracted twice with 10 ml of DCM. The combined organic phases are washed with brine, dried over MgS04 and evaporated. The resulting residue is dissolved in a mixture of 1 ml of methanol and 0.1 ml of acetic acid. The compound is hydrogenated using Pd/C under hydrogen atmosphere. After 20 hour hydrogenation the mixture is filtrated over celite and the filtrate is concentrated under vacuum. The crude is purified using a preparative LC/MS resulting in 2.3 mg of product **53**. (yield=7.4%) . ([M+H]+): 516). Ret. Time: 4.45 min (gradient 5 to 85 % acetonitrile in 7 min).

### Example 57: 7-Acetyl-4-[3-(4-chloro-phenoxy)-phenyl]-2,4,6,7,8,9-hexahydro-1,2,7,9-tetraaza-benz[f]inden-5-one; compound with trifluoroacetic acid (not part of the invention)

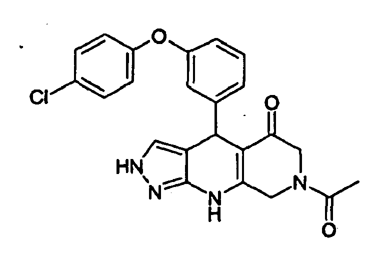

To a solution of 25 mg (0.048 mmole) of compound **48** in 1 ml of DCM are successively added 6 µl of anhydride acetic (0.06 mmole) and 16 µl of DIEA (0.1 mmole). The solution is kept overnight under stirring and poured into 20 ml of water. The mixture is extracted with twice 15 ml of DCM. The combined organic phases are washed with a 10% solution of potassium hydrogenosulfate, water, dried over MgS04 and evaporated. The crude is directly hydrogenated under hydrogen atmosphere using Pd/C as catalyst. The reaction is kept overnight and the reaction mixture is filtrated over celite® (diatomeous earth). The filtrate is concentrated over vacuum and the resulting residue is purified using a preparative LC/MS. 1.1 mg of desired product 57 is isolated (yield= 4%). ([M+H]+): 435). Ret. Time: 3.71 min (gradient 5 to 85 % acetonitrile in 7 min).

### Example 58: 9-Acetyl-4-[3-(4-chloro-phenoxy)-phenyl]-2,4,6,7,8,9-hexahydro-1,2,7,9-tetraaza-benz[f]inden-5-one; compound with trifluoroacetic acid

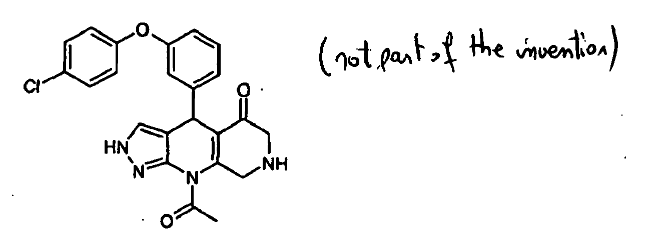

To a solution of 25 mg (0.048 mmole) of compound **46** in 0.5 ml of DCM are successively added 11 ILl of anhydride acetic (0.11 mmole) and 33 µl of DIEA (0.2 mmole). The solution is stirred at room temperature for 2 hours and poured into 20 ml of water. The mixture is extracted with twice 15 ml of DCM. The combined organic phases are washed with a 10% solution of potassium hydrogenosulfate, water, dried over MgS04 and evaporated. The crude is directly treated with 1 ml of TFA/DCM (50/50) solution at room temperature for 1 hour. The mixture is concentrated under vacuum and the resulting residue is purified using a preparative LC/MS. 23 mg of desired product **58** is isolated (yield= 52%). ([M+H]+): 435). Ret. Time: 4.33 min (gradient 5 to 85 % acetonitrile in 7 min).

### Example 59: 4-[5-(1H-Benzimidazol-2-ylsulfanyl)-furan-2-yl]-9-methyl-2,4,6,7,8,9-hexahydro-pyrazolo[3,4-b] quinolin-5-one; compound with trifluoro-acetic acid

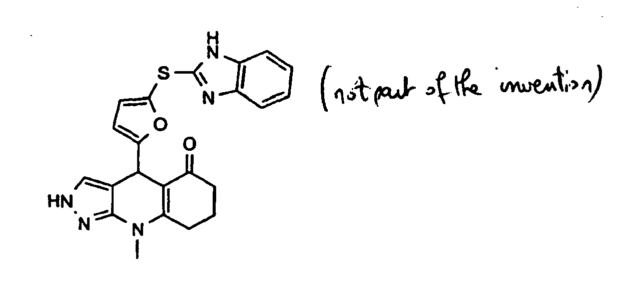

To a solution of 30 mg (0.075 mmole) of compound **3** and 14 µl of methyl iodide (0.225 mmole)in 1 ml of DMF is added 31 mg of potassium carbonate (0.225 mmole). The solution is stirred for 20 hours at room temperature and poured into 20 ml of water. The mixture is extracted with twice 15 ml of DCM. The combined organic phases are washed with brine, dried over MgS04 and evaporated. The crude is purified using a preparative LC/MS. 2.2 mg of desired product **59** is isolated (yield= 6%). ([M+H]+): 418). Ret. Time: 2.54 min (gradient 5 to 85 % acetonitrile in 7 min).

### Example 60: 3-(5-Oxo-4,5,6,7,8,9-hexahydro-2H-1,2,7,9-tetraazabenz[f]inden-4-yl)-N-(4-trifluoromethoxy-benzyl)-benzamide; compound with trifluoro-acetic acid

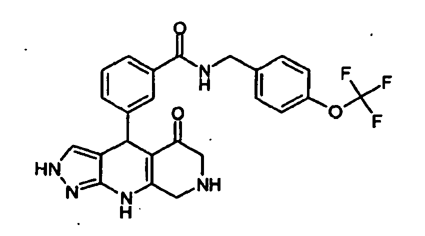

Preparation of the aldehyde: to a solution of 300 mg of 3-carboxybenzaldehyde (2 mmoles) and 382 mg of 4-(trifluoromethoxy)benzylamine (2 mmoles) in 5 ml of DCM is successively added 540 mg of 1-hydroxybenzotriazole (HOBt) (4 mmoles) and 0.63 mg of diisopropylcarbodiimide (DIC) (4 mmoles). The reaction mixture is stirred overnight at room temperature and then poured into 20 ml of 10% KH₂SO₄ solution. The mixture is extracted with twice 15 ml of ethyl acetate. The combined organic phases are washed with 20 ml of water, 20 ml of brine, dried over MgSO₄ and evaporated giving 670 mg of 3-formyl-N-(4-trifluoromethoxy-benzyl)-benzamide (yield=85%). ([M+H]+): 324). Ret. Time: 5.24 min (gradient 5 to 85 % acetonitrile in 7 min). The compound **60** is prepared as described for example **2** starting with 21.3 mg of of N-Boc-3, 4-diketopiperidine (0.1 mmole), 8.3 mg aminopyrazole (0.1 mmole) and 32.3 mg of aldehyde (0.1 mmole) in a mixture of 0.5ml of ethanol and 0.5 ml of DMF resulting in 22.5 mg of product **60** after purification by preparative LC/MS. (yield= 38%). ([M+H]+): 484). Ret. Time: 2.51 min (gradient 2 to 80 % acetonitrile in 7 min).

### Example 61: 3-(5-Oxo-4,5,6,7,8,9-hexahydro-2H-1,2,7,9-tetraazabenz[f]inden-4-yl)-N-(3-trifluoromothoxy-benzyl)-benzamide; compound with trifluoro-acetic acid

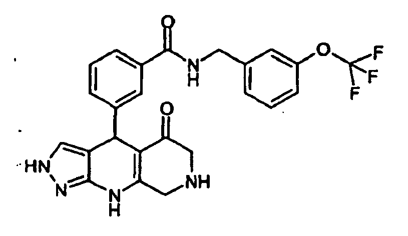

Compound **61** was prepared using the same procedure as described for **60** starting with 3-(trifluoromethoxy) benzylamine. 25.5 mg of desired compound was isolated after preparative LC/MS (yield= 43%). ([M+H]+): 484). Ret. Time: 2.47 min (gradient 2 to 80 % acetonitrile in 7 min).

### Example 62: 3-(5-Oxo-4,5,8,7,8,9-hexahydro-2H-1,2,7,9-tetraazabenz[f]inden-4-yl)-N-(4-trifluoromethoxy-phenyl)-banzamide; compound with trifluoro-acetic acid

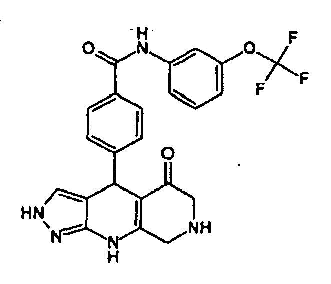

Preparation of the aldehyde: to a solution of 300 mg of 4-carboxybenzaldehyde (2 mmoles) and 382 mg of 4-(trifluoromethoxy)aniline (2 mmoles) in 5 ml of ethyl acetate is added 830 mg of dicyclohexycarbodiimide) (DCC) (4 mmoles). The reaction mixture is heated at 60°C overnight and then poured into 60 ml of 1 N HCl solution. The mixture is extracted with twice 30 ml of ethyl acetate. The combined organic phases are washed with 50 ml of water; 50 ml saturated solution of sodium bicarbonate and brine, dried over MgSO₄ and evaporated. The crude is purified on silica gel giving 233 mg of 4-Formyl-N-(3-trifluoromethoxy-phenyl)-benzamide (yield=38%). ([M+H]+): 310). Ret. Time: 5.60 min (gradient 5 to 85 % acetonitrile in 7 min).

Starting with 30.9 mg of aldehyde (0.1 mmole) and using the same procedure as example **2,** 22.3 mg of the product **62** was obtained after preparative LC/MS (yield= 43%). ([M+H]+): 470). Ret. Time: 2.61 min (gradient 2 to 80 % acetonitrile in 7 min).

The following examples were obtained using the same procedure:

**Table 4**

| Examples | Chemistry | MW parent compound | EIMS ([M+H]⁺ | RT (min) |
|---|---|---|---|---|
| 63 | 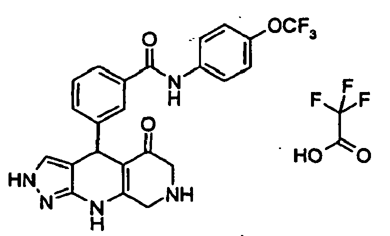 | 469.43 | 470 | 2.57 |
| 64 | 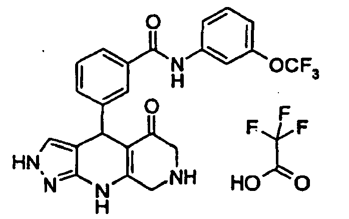 | 469.43 | 470 | 2.58 |
| LC/MS conditions: gradient 2 to 80 % acetonitrile in 7 min | | | | |

### Example 65: 4-Chloro-N-[3-(5-oxo-4,5,6,7,8,9-hexahydro-2H-1,2,7,9-tetraaza-benz[f]inden-4-yl)-phenyl]-benzamide; compound with trifluoroacetic acid

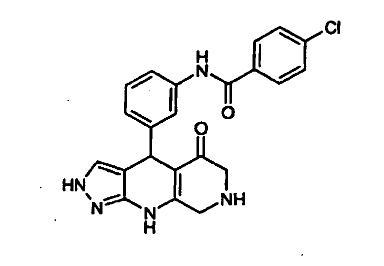

Preparation of the aldehyde: to a solution of 242 mg of 3-aminobenzaldehyde (2 mmoles) and 250 µl of 4-chlorobenzoyl chloride (2 mmoles) in 5 ml of DMF is added 700 µl of DIEA(4 mmoles). The reaction is heated in a microwave at 110°C for 10 minutes. The precipitate formed is filtrated and washed with methanol. 670mg of 4-Chloro-N-(3-formyl-phenyl)-benzamide as a white solid is isolated (yield=85%). ([M+H]+): 260

Starting with 26 mg of aldehyde (0.1 mmole) and using the same procedure as for example 2, 25 mg of the product **65** was obtained after preparative LC/MS (yield= 53%). ([M+H]+): 420). Ret. Time: 2.14 min (gradient 2 to 80 % acetonitrile in 7 min).

### Example 66: 4-Chloro-N-[5-(5-oxo-4,5,6,7,8,9-hexahydro-2H-1,2,7,9-tetraaza-benz[f]inden-4-yl)-thiazol2-yl]-benzamlde; compound with trifluoro-acetic acid

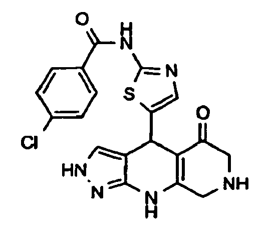

Compound **66** was prepared using the same procedure as described for **65**. 266 mg of 4-chloro-N-(5-formylthiazol-2-yl)-benzamide were prepared from 256 mg of 2-amino-thiazole-5-carbaldehyde (2 mmoles) and 250 µl of 4-chlorobenzoyl chloride (2 mmoles) (yield= 26%). ([M+H]+): 282) Ret. Time: 6.10 min (gradient 2 to 80 % acetonitrile in 7 min).. Starting from 27 mg of aldehyde (0.1 mmole), 15.2 mg of desired compound **66** was isolated after purification by preparative LC/MS (yield= 28%). ([M+H]+): 427). Ret. Time: 2.53 min (gradient 2 to 80 % acetonitrile in 7 min).

### Example 67: 4-Chloro-N-[5-(5-oxo-4,5,6,7,8,9-hexahydro-2H-1,2,7,9-tetraaza-benz[f]inden-4-yl)-thiazol-2-yl]-benzamide; compound with trifluoro-acetic acid

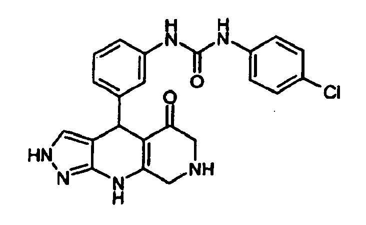

Preparation of the aldehyde: a mixture of 360 mg of 3-aminobenzaldehyde (0.3 mmoles) and 460 mg of 4-chloro-phenylisocyanate (0.3 mmoles) in 3 ml of DMF is heated in a microwave at 110°C for 10 minutes. The precipitate formed is filtrated giving the desired 1-(4-Chloro-phenyl)-3-(3-formyl-phenyl)-urea. ([M+H]+): 351. Ret. Time: 5.30 min (gradient 5 to 85 % acetonitrile in 7 min).

Starting with 27 mg of aldehyde (0.1 mmole) and using the same procedure as example 2, 23 mg of the product **67** was obtained after preparative LC/MS (yield= 42%). ([M+H]+): 435). Ret. Time: 2.26 min (gradient 2 to 80 % acetonitrile in 7 min).

### Example 68: 4-(5-Oxo-4,5,6,7,8,9-hexahydro-2H-1,2,7,9-tetraazabenz[f]inden-4-yl)-N-(4-trifluoromethoxy-phenyl)-benzenesulfonamide; compound with trifluoro-acetic acid

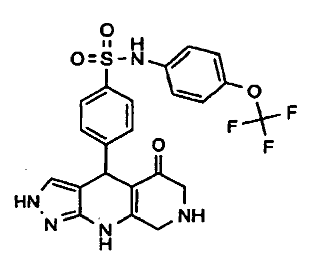

Preparation of the aldehyde: to a mixture of 610 mg of 4-chlorosulfonylbenzaldehyde (3 mmoles) and 460 mg of 4-trifluoromethoxyaniline (3 mmoles) in 2 ml of dichloroethane is added 2 ml of pyridine (25 mmoles). The reaction mixture is stirred at room temperature for 5 hours and then poured into 100ml of 10% HCl solution. The mixture is extracted with twice 30 ml of DCM. The combined organic phases are washed with 30 ml of water; 30 ml of saturated solution of sodium bicarbonate and brine, the solution is dried over MgS04 and evaporated. The crude is purified on silica gel using DCM/AcOEt 90/10 as eluent. 0.48g of 4-formyl-N-(4-trifluoromethoxy-phenyl)-benzenesulfonamide is isolated as a white solid (yield=47%). ([M+H]+): 346. Ret. Time: 5.53 min (gradient 5 to 85 % acetonitrile in 7 min).

Starting with 34.5 mg of aldehyde (0.1 mmole) and using the same procedure as example **2**, 19 mg of the product **68** was obtained after preparative LC/MS (yield= 31%). ([M+H]+): 506). Ret. Time: 2.56 min (gradient 2 to 80 % acetonitrile in 7 min).

### Example 69: N-(4-Chloro-phenyl)-4-(5-oxo-4,5,8,7,8,9-hexahydro-2H-1,2,7,9-tetraaza-benz[f]inden-4-yl)-benzenesulfonamide; compound with trifluoro-acetic acid

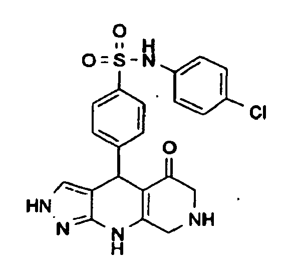

**69** was prepared using the same procedure as described for **68.** 450 mg of N-(4-Chloro-phenyl)-4-formylbenzenesulfonamide were prepared from 256 mg of 4-chlorosulfonylbenzaldehyde (3 mmoles) and 250 ul of 4-chlorobenzoyl chloride (3 mmoles) (yield= 51%). ([M+H]+): 296). Ret. Time: 5.20 min (gradient 5 to 85 % acetonitrile in 7 min). Starting from 29.6 mg of aldehyde (0.1 mmole), 18.8 mg of desired compound **71** was isolated after purification by preparative LC/MS (yield= 33%). ([M+H]+): 456). Ret. Time: 2.28 min (gradient 2 to 80 % acetonitrile in 7 min).

### Example 70: 4-[5-(5-Methyl-1H-benzimidazol-2-ylsulfanyl)-furan-2-yl]-2,4,6,7,8,9-hexahydro-pyrazolo[3,4-b] quinolin-5-one; compound with trifluoro-acetic acid; compound with trifluoro-acetic acid

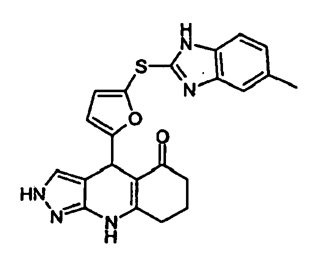

Preparation of the aldehyde: to a solution of 0.82 g of 2-mercapto-5-methyl benzimidazole (5 mmoles) in 10 ml of dry THF is added 200mg of sodium hydride suspension (60% suspension in mineral oil) (5 mmoles). The reaction mixture is refluxed for ½ hour. The mixture is cooled to room temperature and 0.71g of 5-nitrofuraldehyde (5 mmoles) in 5 ml of THF is added dropwise. The reaction mixture is stirred during ½ hour and then poured into 200 ml of water. The mixture is extracted with twice 75 ml of EtOAc. The combined organic phases are washed with brine, dried over MgS04 and evaporated. The crude is purified on silica gel using DCM/MeOH 97/3 as eluent. 1.07 g of 5-(6-Methyl-1H-benzimidazol-2-ylsulfanyl)-furan-2-carbaldehyde is isolated as a black glassy solid (yield=83%). ([M+H]+): 259. Ret. Time: 3.64 min (gradient 0 to 50 % acetonitrile in 7 min).

Starting with 51.7 mg of aldehyde (0.2 mmole) and using the same procedure as example **3**, 48.5 mg of the product **70** was obtained after preparative LC/MS (yield= 46%). ([M+H]+): 418). Ret. Time: 2.48 min (gradient 2 to 80 % acetonitrile in 7 min).

The following examples were obtained using the same procedure:

**Table 5**

| Examples | Chemistry | MW parent compound | EIMS ([M+H]⁺ | RT (min) |
|---|---|---|---|---|
| 71 | 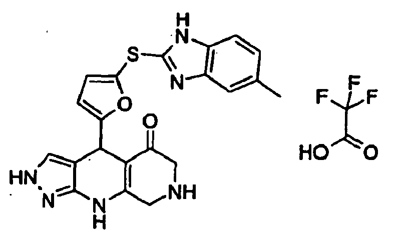 | 532.50 | 533 | 2.48 |
| 72 | 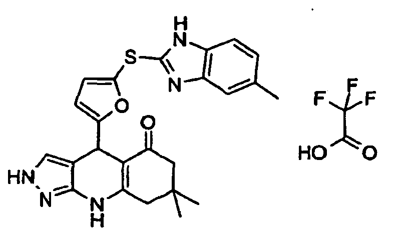 | 559.57 | 560 | 3.39 |
| LC/MS conditions: gradient 2 to 80 % acetonitrile in 7 min | | | | |

### Example 73: 4-[5-(5-Chloro-benzothiazol-2-ylsulfanyl)-furan-2-yl]-2,4,6,7,8,9-hexahydro-1,2,7,9-tetraaza-benz [f]inden-5-one; compound with trifluoro-acetic acid; compound with trifluoro-acetic acid

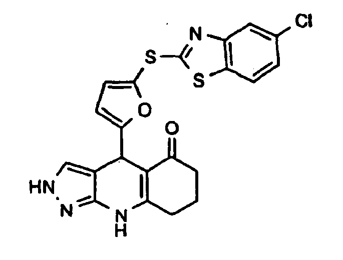

304 mg of 5-(5-Chloro-benzothiazol-2-ylsulfanyl)-furan-2-carbaldehyde as yellow solid was obtained using the same procedure as described in example **72** starting from 402 mg of 5-chloro-2-mercaptobenzothiazole (2 mmoles) and 282 mg of 5-nitro-2-furaldehyde (2 mmoles) and 80 mg of NaH (Yield=52%)). ([M+H]+): 296). Ret. Time: 3.47 min (gradient 30 to 90 % acetonitrile in 7 min).

Starting with 50 mg of aldehyde (0.17 mmole) and using the same procedure as example **3**, 2.6 mg of the product 73 was obtained after preparative LC/MS (yield= 3%). ([M+H]+): 455). Ret. Time: 3.97 min (gradient 2 to 80 % acetonitrile in 7 min).

The following examples were obtained using the same procedure:

**Table 6**

| Examples | Chemistry | MW parent compound | EIMS ([M+H]⁺ | RT (min) |
|---|---|---|---|---|
| 74 | 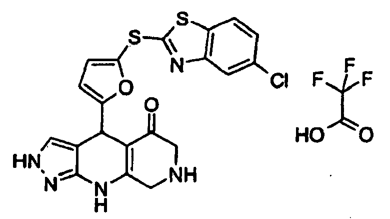 | 569.97 | 570 | 3.81 |
| 75 | 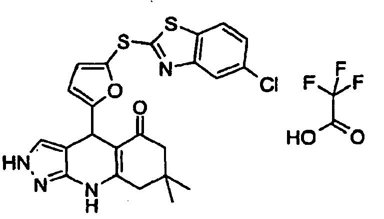 | 597.04 | 598 | 4.86 |
| LC/MS conditions: gradient 2 to 80 % acetonitrile in 7 min | | | | |

### Example 76: 4-[5-(5-Difluoromethoxy-1H-benzimidazol-2-ylsulfanyl)-furan-2-yl]-2,4,6,7,8,9-hexahydro-pyrazolo[3,4-b]quinolin-5-one; compound with trifluoro-acetic acid

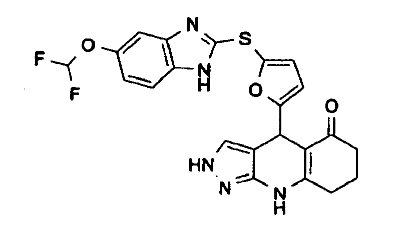

417 mg of 5-(5-difluoromethoxy-1H-benzimidazol-2-ylsulfanyl)-furan-2-carbaldehyde was obtained using the same procedure as described in example **70** using 1.08 g of 5-difluoromethoxy-2-mercaptobenzimidazole (5 mmoles ) and 0.71 g of 5- nitro-2-furaldehyde (5 mmoles) and 200 mg of NaH (Yield=27%). ([M+H]+): 311). Ret. Time: 4.0 min (gradient 0 to 50% acetonitrile in 7 min).

Starting with 63 mg of aldehyde (0.2 mmole) and using the same procedure as example **3,** 10 mg of the product **76** was obtained after preparative LC/MS (yield= 9%). ([M+H]+): 470). Ret. Time: 3.15 min (gradient 5 to 85 % acetonitrile in 7 min).

### Example 77: 4-[5-(6-Methoxy-1H-benzimidazol-2-yloxy)-furan-2-yl]-2,4,6,7,8,9-hexahydro-pyrazolo[3,4-b]quinolin-5-one; compound with trifluoro-acetic acid

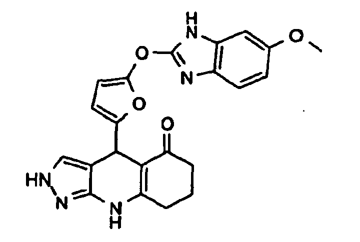

15 mg of 5-(6-Methoxy-1H-benzimidazol-2-yloxy)-furan-2-carbaldehyde was obtained using the same procedure as described in example **70** using 0.82 g of 5-methoxy-2-benzimidazolinone (5 mmoles) and 0.71 g of 5- nitro-2-furaldehyde (5 mmoles) and 200 mg of NaH (Yield=1.2%). ([M+H]+): 259). Ret. Time: 2.53 min (gradient 5 to 85% acetonitrile in 7 min).

Starting with 15 mg of aldehyde (0.06 mmole) and using the same procedure as example **3**, 2.5 mg of the product **77** was obtained after preparative LC/MS (yield= 8%). ([M+H]+): 418). Ret. Time: 1.03 min (gradient 10 to 95 % acetonitrile in 7 min).

### Example 78: 4-[5-(4-methyl-1H-imidazol-2-ylsulfanyl)-furan-2-yl]-2,4,6,7,8,9-hexahydro-pyrazolo[3,4-b]quinolin-5-one; compound with trifluoro-acetic acid

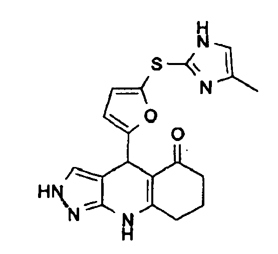

1.84 g of 5-(4-Methyl-1H-imidazol-2-ylsulfanyl)-furan-2-carbaldehyde was obtained using the same procedure as described in example **70** using 1 g of 4-methyl-1 H-2-mercaptoimidazole (8.75 mmoles) and 1.23 g of 5- nitro-2-furaldehyde (8.75 mmoles) and 350 mg of NaH (Yield=100%). ([M+H]+): 209). Ret. Time: 0.53 min (gradient 0 to 50% acetonitrile in 7 min).

Starting with 41.7 mg of aldehyde (0.2 mmole) and using the same procedure as example **3**, 56 mg of the product **78** was obtained after preparative LC/MS (yield= 58%). ([M+H]+): 368). Ret. Time: 2.15 min (gradient 2 to 80 % acetonitrile in 7 min).

### Example 79: 4-15-(5-Methoxy-1H-benzimidazol-2-ylsulfanyl)-furan-2-yl]-2,4,6,7,8,9-hexahydro-pyrazolo[3,4-b] quinolin-5-ons; compound with trifluoro-acetic acid

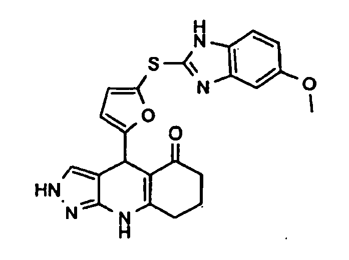

1.8 g of 5-(6-methoxy-1H-benzimidazol-2-ylsulfanyl)-furan-2-carbaldehyde was obtained using the same procedure as described in example **70** using 1.8 g of 5-methoxy-2-mercaptobenzimidazole (10 mmoles) and 1.41 g of 5-nitro-2-furaldehyde (10 mmoles) and 400 mg of NaH (Yield=66%). ([M+H]+): 274). Ret. Time: 4.13 min (gradient 0 to 50% acetonitrile in 7 min).

Starting with 54.9 mg of aldehyde (0.2 mmole) and using the same procedure as example **3,** 45.1mg of the product **79** was obtained after preparative LC/MS (yield= 41%). ([M+H]+): 434). Ret. Time: 2.93 min (gradient 2 to 80 % acetonitrile in 7 min).

The following examples were obtained using the same procedure:

**Table 7**

| Examples | Chemistry | same procedure MW parent compound | EIMS ([M+H]⁺ | RT (min) |
|---|---|---|---|---|
| 80 | 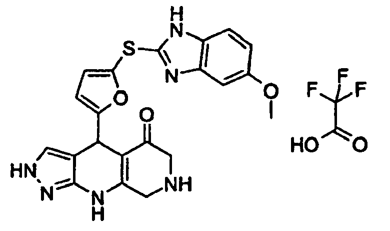 | 434.00 | 435 | 2.34 |
| 81 | 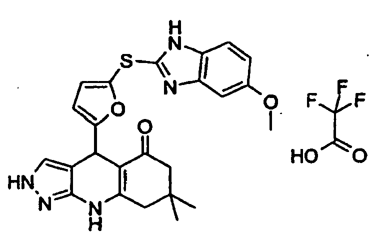 | 461.00 | 462 | 3.26 |
| LC/MS conditions: gradient 2 to 80 % acetonitrile in 7 min | | | | |

### Example 82: 4-[5-(1-Methyl-1H-benzimidazol-2-yisulfanyl)-furan-2-yl]-2,4,6,7,8,9-hexahydro-pyrazolo[3,4-b] quinolin-5-one; compound with trifluoro-acetic acid

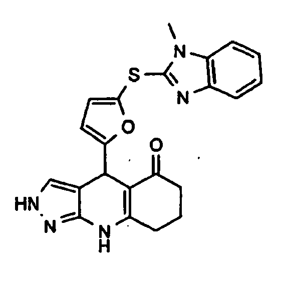

1.52 g of 5-(1-Methyl-1H-benzimidazol-2-ylsulfanyl)-furan-2-carbaldehyde was obtained using the same procedure as described in example **70** using 0.82 g of 2-mercapto-5-methylbenzimidazole (5 mmoles) and 0.71 g of 5-nitro-2-furaldehyde (5 mmoles) and 200 mg of NaH (Yield=85%). ([M+H]+): 259). Ret. Time: 1.64 min (gradient 2 to 85% acetonitrile in 7 min).

Starting with 51.7 mg of aldehyde (0.2 mmole) and using the same procedure as example **3,** 53.3 mg of the product **82** was obtained after preparative LC/MS (yield= 50%). ([M+H]+): 418). Ret. Time: 2.74 min (gradient 2 to 80 % acetonitrile in 7 min).

The following examples were obtained using the same procedure:

**Table 8**

| Examples | Chemistry | MW parent compound | EIMS ([M+H]⁺ | RT (min) |
|---|---|---|---|---|
| 83 | 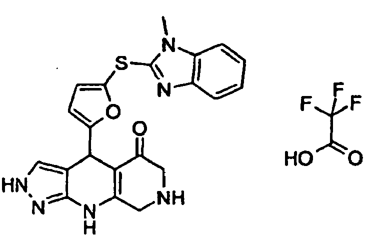 | 418.12 | 419 | 2.19 |
| 84 | 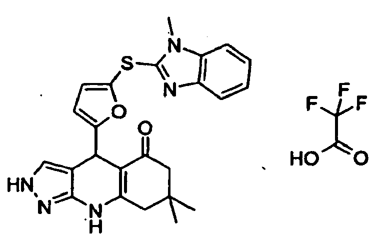 | 445.16 | 446 | 3.63 |
| LC/MS conditions: gradient 2 to 80 % acetonitrile in 7 min | | | | |

### Example 85: 4-[5-(5,6-Dichloro-1H-benzimidazol-2-ylsulfanyl)-furan-2-yl]-2,4,6,7,8,9-hexahydro-pyrazolo [3,4-b]quinolin-5-one; compound with trifluoro-acetic acid

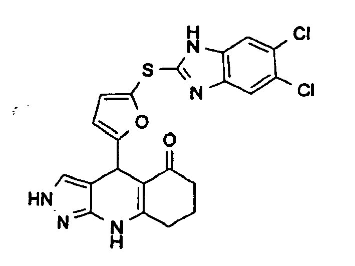

1.24 g of 5-(5,6-Dichloro-1H-benzimidazol-2-ylsulfanyl)-furan-2-carbaldehyde was obtained using the same procedure as described in example **70** using 2.2 g of 5,6-dichloro-2-mercaptobenzimidazole (10 mmoles) and 1.41 g of 5-nitro-2-furaldehyde (10 mmoles) and 400 mg of NaH (Yield=40%). ([M+H]+): 313). Ret. Time: 4.64 min (gradient 0 to 50% acetonitrile in 7 min).

Starting with 62.6 mg of aldehyde (0.2 mmole) and using the same procedure as example **3**, 26 mg of the product **85** was obtained after preparative LC/MS (yield= 19%). ([M+H]+): 472). Ret. Time: 4.13 min (gradient 2 to 80 % acetonitrile in 7 min).

The following examples were obtained using the same procedure:

**Table 9**

| Examples | Chemistry | MW parent compound | EIMS ([M+H]⁺ | RT (min) |
|---|---|---|---|---|
| 86 | 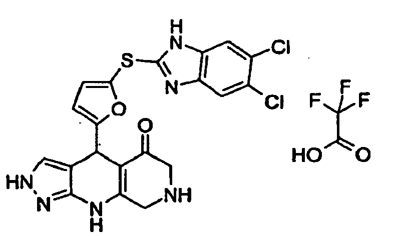 | 472.03 | 473 | 3.46 |
| 87 | 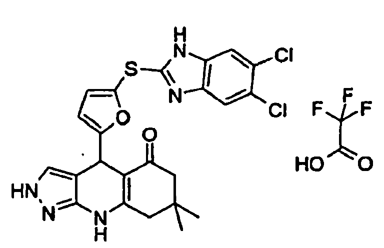 | 499.06 | 500 | 4.49 |
| LC/MS conditions: gradient 2 to 80 % acetonitrile in 7 min | | | | |

### Example 88: 4-[5-(5-Chloro-benzoxazol-2-ylsulfanyl)-furan-2-yl]-2,4,6,7,8,9-hexahydro-pyrazolo[3,4-b]quinolin-5-one; compound with trifluoro-acetic acid

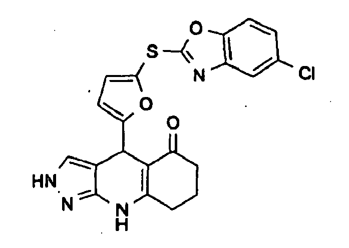

0.58 g of 5-(5-Chloro-benzoxazol-2-ylsulfanyl)-furan-2-carbaldehyde was obtained using the same procedure as described in example **70** using 1.03 g of 5-chloro-2-mercaptobenzoxazole (5.5 mmoles) and 0.78 g of 5- nitro-2-furaldehyde (5.5 mmoles) and 223 mg of NaH (Yield=37%). ([M+H]+): 280). Ret. Time: 4.67 min (gradient 0 to 50% acetonitrile in 7 min).

Starting with 55.9 mg of aldehyde (0.2 mmole) and using the same procedure as example **3**, 10.2 mg of the product **88** was obtained after preparative LC/MS (yield= 9%). ([M+H]+): 439). Ret. Time: 4.25 min (gradient 2 to 80 % acetonitrile in 7 min).

The following examples were obtained using the same procedure:

**Table 10**

| Examples | Chemistry | MW parent compound | EIMS ([M+H]⁺ | RT (min) |
|---|---|---|---|---|
| 89 | 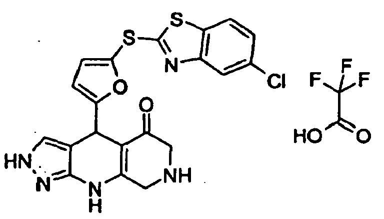 | 455.03 | 456 | 3.81 |
| 90 | 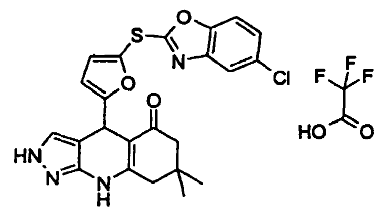 | 466.09 | 467 | 4.62 |
| LC/MS conditions: gradient 2 to 80 % acetonitrile in 7 min | | | | |

### Example 91: 7,7-Dimethyl-4-[5-(4-methyl-1H-imidazol-2-ylsulfanyl)-furan-2-yl]-2,4,6,7,8,9-hexahydro-pyrazolo [3,4-b]quinolin-5-one; compound with trifluoro-acetic acid

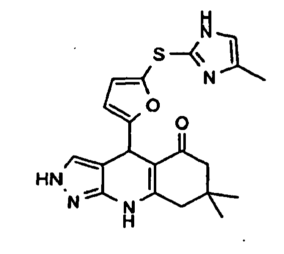

Compound **91** was prepared as **78** starting with 28 mg of dimedone (0.2 mmole). 49.4 of desired compound is isolated after preparative LC/MS (yield=49%). ([M+H]+): 396). Ret. Time: 2.53 min (gradient 2 to 80 % acetonitrile in 7 min).

### Example 92: 4-{5-[5-(4-Chloro-phenyl)-1-methy)-1H-imidazol-2-ylsulfanyl]-furan-2-yl}-2,4,6,7,8,9-hexahydro-pyrazolo[3,4-b]quinolin-5-one

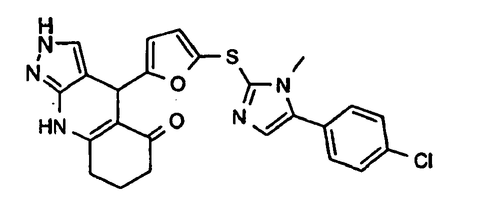

1.5 g of 5-[5-(4-chloro-phenyl)-1-methyl-1H-imidazol-2-ylsulfanyl]-furan-2-carbaldehyde was obtained using the same procedure as described in example **70** staring with 1 g of 1-methyl-5(4-chlorophenyl)imidazol-2-thiol (4.45 mmoles), 0.63 g of 5-nitrofuraldehyde (4.45 mmoles) and 178 mg of sodium hydride (4.45 mmoles). (Yield=100%). ([M+H]+): 319). Ret. Time 3.91 min (gradient 5 to 85% acetonitrile in 7 min).

Starting with 64.2 mg of aldehyde (0.2 mmole) and using the same procedure as example **3**, 56 mg of the product **92** was obtained after chromatography on silica gel using DCM/MeOH 97/3 as eluent (yield= 58%). ([M+H]+): 478). Ret. Time: 3.48 min (gradient 2 to 80 % acetonitrile in 7 min).

The following examples were obtained using the same procedure:

**Table 11**

| Examples | Chemistry | MW parent compound | EIMS ([M+H]⁺ | RT (min) |
|---|---|---|---|---|
| 93 | 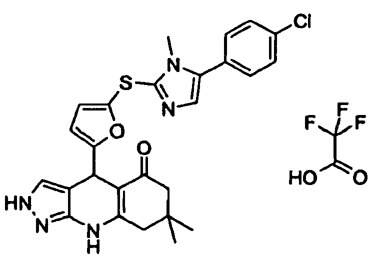 | 505.13 | 506 | 3.74 |
| 94 | 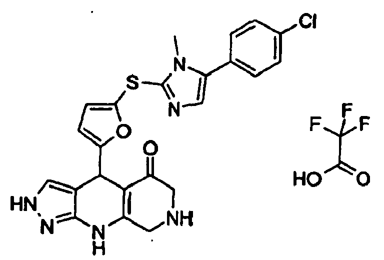 | 478.10 | 479 | 2.87 |
| LC/MS conditions: gradient 2 to 80 % acetonitrile in 7 min | | | | |

### Example 95: 4-[5-(5-Trifluoromethyl-1H-benzimidazol-2-ylsulfanyl)-furan-2-yl]-2,4,6,7,8,9-hexahydro-pyrazolo [3,4-b]quinolin-5-one

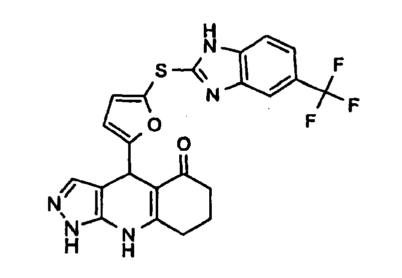

1.4 g of 5-(5-Trifluoromethyl-1 H-benzimidazol-2-ylsulfanyl)-furan-2-carbaldehyde was obtained using the same procedure as described in example **70** staring with 1 g of 2-mercapto-5-trifluoromethylbenzimidazole (4.6 mmoles), 0.65 g of 5-nitrofuraldehyde (4.6 mmoles) and 180 mg of sodium hydride (4.6 mmoles). (Yield=99%). ([M+H]+): 313). Ret. Time 4.78 min (gradient 5 to 85% acetonitrile in 7 min).

Starting with 63 mg of aldehyde (0.2 mmole) and using the same procedure as example **3**, 43 mg of the product **95** was obtained after chromatography on silica gel using DCM/MeOH 97/3 as eluent (yield= 46%). ([M+H]+): 472). Ret. Time: 3.84 min (gradient 2 to 80 % acetonitrile in 7 min).

The following examples were obtained using the same procedure:

**Table12**

| Examples | Chemistry | MW parent compound | EIMS ([M+H]⁺ | RT (min) |
|---|---|---|---|---|
| 96 | 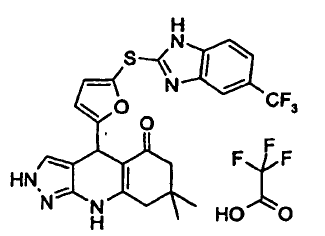 | 499.52 | 500 | 4.22 |
| 97 | 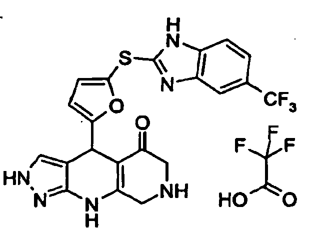 | 472.09 | 473 | 3.25 |
| LC/MS conditions: gradient 2 to 80 % acetonitrile in 7 min | | | | |

### Example 98: 4-[5-(4,5-Dimethyl-1H-imidazol-2-ylsulfanyl)-furan-2-yl]-2,4,6,7,8,9-hexahydro-pyrazolo[3,4-b]quinolin-5-one

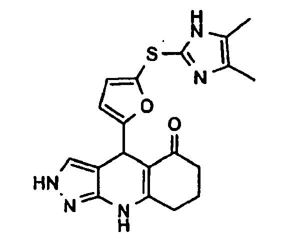

2 g of 5-(4,5-Dimethyl-1H-imidazol-2-ylsulfanyl)-furan-2-carbaldehyde was obtained using the same procedure as described in example **70** starting with 1 g of 4,5-dimethyl-2-mercaptoimidazole (7.96 mmoles), 1.12 g of 5-nitrofuraldehyde (7.96 mmoles) and 320 mg of sodium hydride (7.96 mmoles). (Yield=100%). ([M+H]+): 223). Ret. Time 2.34 min (gradient 5 to 85% acetonitrile in 7 min).

Starting with 44.5 mg of aldehyde (0.2 mmole) and using the same procedure as example **3**, 36.4mg of the product **98** was obtained after chromatography on silica gel using DCM/MeOH 97/3 as eluent (yield= 37%). ([M+H]+): 382). Ret. Time: 2.31 min (gradient 2 to 80 % acetonitrile in 7 min).

The following examples were obtained using the same procedure:

**Table 13**

| Examples | Chemistry | MW parent compound | EIMS ([M+H]⁺ | RT (min) |
|---|---|---|---|---|
| 99 | 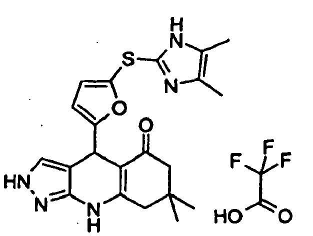 | 409.51 | 410 | 2.69 |
| 100 | 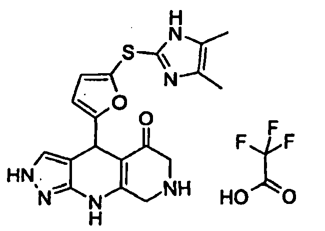 | 382.45 | 383 | 1.77 |
| LC/MS conditions: gradient 2 to 80 % acetonitrile in 7 min | | | | |

### Example 101:4-[5-(5-methyl-1H-benzimidazol-2-ylsulfanyl)-thiophen-2-yl]-2,4,6,7,8,9-hexahydro-pyrazolo [3,4-b]quinolin-5-one

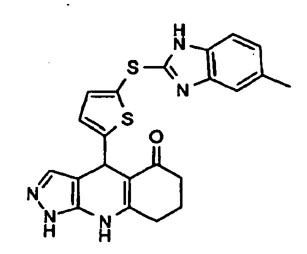

Preparation of the aldehyde: a mixture of 0.82 g 2-mercapto-5-methylbenzimidazol (5 mmoles), 0.6 ml of 5-bromo-2-thiophenecarboxaldehyde (5 mmoles) and 1.4 g of potassium carbonate in 10 ml of DMF is heated at 110°C for 2 hours. The mixture is cooled down at room temperature then poured into 200 ml of water and extracted with twice 50 ml of ethyl acetate. The combined organic phases are washed with brine, dried over MgS04 and evaporated. The crude is purified on silica gel using DCM/MeOH as eluent giving 0.65 g of 5-(5-methyl-1H-benzimidazol-2-ylsulfanyl)-thiophene-2-carbaldehyde. (Yield= 47%). ([M+H]+): 275). Ret. Time 3.42 min (gradient 5 to 85% acetonitrile in 7 min).

Starting with 54.9 mg of aldehyde (0.2 mmole) and using the same procedure as example 3, 78.8 mg of the product 101 was obtained after chromatography on silica gel using DCM/MeOH 97/3 as eluent (yield= 91%). ([M+H]+): 434). Ret. Time: 3.07 min (gradient 2 to 80 % acetonitrile in 7 min).

The following examples were obtained using the same procedure:

**Table 14**

| Examples | Chemistry | MW parent compound | EIMS ([M+H]⁺ | RT (min) |
|---|---|---|---|---|
| 102 | 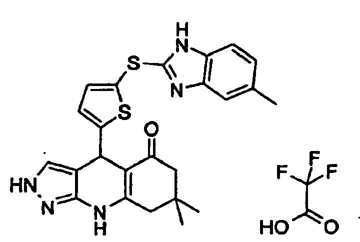 | 461.61 | 462 | 3.44 |
| 103 | 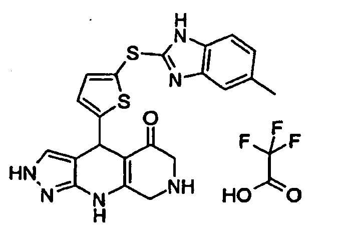 | 434.10 | 435 | 2.5 |
| LC/MS conditions: gradient 2 to 80 % acetonitrile in 7 min | | | | |

### Example 104: 9-[5-(1H-Benzimidazol-2-ylsulfanyl)-furan-2-yl]-8-oxo-4,5,6,7,8,9-hexahydro-2H-pyrrolo[3,4-b] [1,7]naphthyridine-3-carboxylic acid ethyl ester; compound with trifluoro-acetic acid

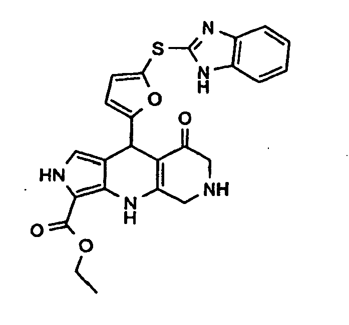

The compound **104** was obtained using the same procedure as example **2** using 42 mg of N-Boc-3, 4-diketopiperidine (0.2 mmole), 38 mg of 3-amino-ethoxycarbonylpyrrol hydrochloride (0.2 mmole), 51 mg of 5-(1H-benzimidazol-2-ylsulfanyl)-furan-2-carbaldehyde (0.2 mmole) and 33 µl of DIEA (0.2 mmole). After preparative LC/MS 8.5 mg of desired product 104 was isolated (yield= 7%). ([M+H]+): 476). Ret. Time: 2.98 min (gradient 5 to 85 % acetonitrile in 7 min).

### Example 105: 9-[5-(1H-Benzimidazol-2-ylsulfanyl)-furan-2-yl]-8-oxo-4,5,8,7,8,9-hexahydro-2H-pyrrolo[3,4-b] quinoline-3-carboxylic acid ethyl ester; compound with trifluoro-acetic acid

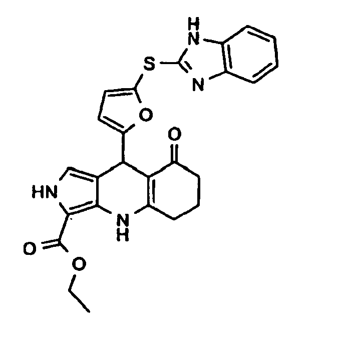

The compound **105** was obtained using the same procedure as example **3** using 22.4 mg of 1,3-cyclohexanedione (0.2 mmole), 38 mg of 3-amino-ethoxycarbonylpyrrol hydrochloride (0.2 mmole), 51 mg of 5-(1 H-benzimidazol-2-ylsulfanyl)-furan-2-carbaldehyde (0.2 mmole) and 33 µl of DIEA (0.2 mmole). After preparative LC/MS 7.7 mg of desired product **105** was isolated (yield= 7%). ([M+H]+): 475). Ret. Time: 3.94 min (gradient 5 to 85 % acetonitrile in 7 min).

The following examples were obtained using the same procedure:

**Table 15**

| Examples | Chemistry | MW parent compound | EIMS ([M+H]⁺ | RT (min) |
|---|---|---|---|---|
| 106 | 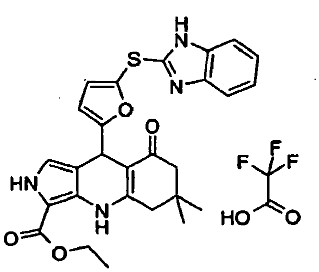 | 502.59 | 503 | 4.3 |
| 107 | 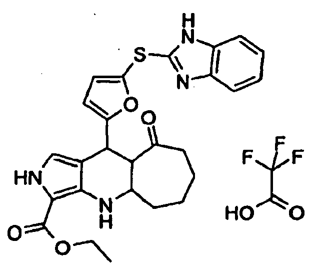 | 488.57 | 489 | 4.69 |
| LC/MS conditions: gradient 5 to 85 % acetonitrile in 7 min | | | | |

### Example 108: 9-[5-(5-Methyl-1H-benzimidazol-2-ylsulfanyl)-furan-2-yl]-8-oxo-4,5,6,7,8,9-hexahydro-2H-pyrrolo[3,4-b]quinoline-3-carboxylic acid ethyl ester

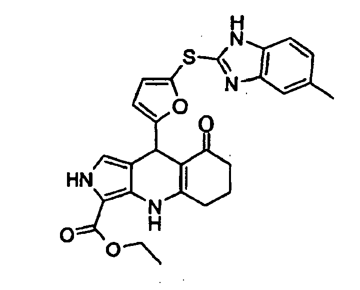

Step 1: 5-(5-Methyl-1H-benzimidazol-2-ylsulfanyl)-furan-2-carbaldehyde 1.16 g of 2-mercapto-5-methylbenzimidazole in 14 mL of tetrahydrofurane (THF) is dropped into a 100 mL three-neck flask, and then 309 mg of sodium hydride is added. The mixture is stirred at reflux temperature during 30 minutes followed by addition of 1 g of 5-nitro furaldehyde in 7 mL of THF. The reaction medium is allowed to cool down to room temperature (ca. 20°C), and then poured in a mixture of 200 mL of water and 100 mL of ethyl acetate (EtOAc). The organic layer is isolated and the aqueous layer is extracted twice with EtOAc (2 x 100 mL). The organic layers are joined together, dried under magnesium sulfate, and evaporated under reduced pressure. 1.8 g of 5-(5-Methyl-1H-benzoimidazol-2-ylsulfanyl)-furan-2-carbaldehyde is collected (RT 2.98 min; [M+H]⁺ : 259; 80% UV)

### Step 2: 9-[5-(5-Methyl-1H-benzoimidazol-2-ylsulfanyl)-furan-2-yl]-8-oxo-4,5,6,7,8,9-hexahydro-2H-pyrrolo[3,4-b]quinoline-3-carboxylic acid ethyl ester (title compound)

774 µmol of 3-amino-2-ethoxycarbonylpyrrole hydrochloride, 774 µmol of 5-(5-Methyl-1H-benzoimidazol-2-ylsulfanyl)-furan-2-carbaldehyde (obtained from step 1), 774 µmol of 1,3 cyclohexanedione and 400 µL of N,N-diisopropylethylamine in 3 mL of ethanol are poured into a flask suitable for microwave irradiation (Personal synthesizer). The flask is locked then irradiation is performed at 100°C during 700 seconds. After cooling down to 20°C, the reaction mixture is evaporated under reduced pressure. The remaining crude is purified on silica gel (Automate Intelliflash, Analogix SiO₂ 75g; Eluent EtOAc / cyclohexane ; from 10/90 to 80/20.(v/v), rate : 25mUmin). Fractions containing the expected compound are joined and evaporated under reduced pressure. The remaining oil is solubilized in 0.5 mL of dichloromethane (DCM) then crystallized by addition of small amounts of diisopropyloxide. 60 mg of solid is collected. 400 MHz ¹H NMR spectrum recorded on BRUKER AVANCE DRX-400 spectrometer. Solvent: DMSO-d6, (DMSO signal locked at 2.50 ppm); δ (ppm) : 1,28 (t, J = 7,0 Hz, 3H) ; 1,89 (m, 2H) ; 2,25 (m, 2H) ; 2,38 (s, 3H) ; 2,57 (m, 1H) ; 2,79 (m, 1 H) ; 4,26 (q, J = 7,0 Hz, 2H) ; 5,14 (s, 1 H) ; 5,96 (d, J = 3,0 Hz, 1 H) ; 6,80 (d large, J = 3,0 Hz, 2H) ; 6,98 (m large, 1 H) ; 7,15 - 7,45 (m large, 2H) ; 8,42 (s, 1 H) ; 11,40 (m large, 1 H) ; 12,3 (m large, 1 H).
MS : ES , m/z=489 = MH⁺.

### Example 109:

### 6,6-Dimethyl-9-[5-(5-methyl-1H-benzimidazol-2-ylsulfanyl)-furan-2-yl]-8-oxo-4,5,6,7,8,9-hexahydro-2H-pyrrolo [3,4-b]quinoline-3-carboxylic acid ethyl ester

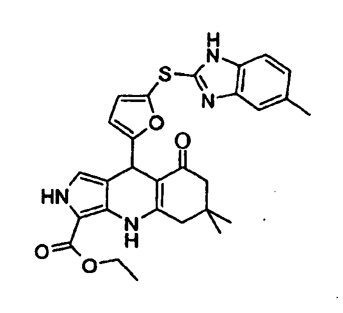

387 µmol of 3-amino-2-ethoxycarbonylpyrrole hydrochloride, 387 µmol of 5-(5-Methyl-1H-benzoimidazol-2-ylsulfanyl)-furan-2-carbaldehyde (obtained from example 108, step 1), 387 µmol of dimedone and 202 µL of N,N-diisopropylethylamine in 1.5 mL of ethanol are poured into a flask suitable for microwave irradiation (Personal synthesizer). The flask is locked then irradiation is performed at 100°C during 700 seconds. After cooling down to 20'C, the reaction mixture is evaporated under reduced pressure. The remaining crude is purified on silica gel (Automate Intelliflash, Analogix SiO₂ 40g; Eluent EtOAc/cyclohexane ; from 30/70 to 75/25.(v/v), rate : 25mUmin, during 70 minutes). Fractions containing the expected compound are joined and evaporated under reduced pressure. The remaining oil is solubilized in 0.5 mL of dichloromethane (DCM) then crystallized by addition of small amounts of diisopropyloxide. 62 mg of expected compound as a solid is collected.
300 MHz ¹H NMR spectrum recorded on BRUKER AVANCE DPX-300 spectrometer. Solvent: DMSO-d6, (DMSO signal locked at 2.50 ppm); δ (ppm):

The following signals are attributed to one species (abundance 75 % over the spectrum) : 0,92 (s, 3H) ; 1,00 (s, 3H) ; 1,29 (t, J = 7,0 Hz, 3H) ; 2,05 (d, J = 16,5 Hz, 1 H) ; 2,18 (d, J = 16,5 Hz, 1 H) ; 2,38 (s, 3H) ; 2,45 - 2,65 (m hidden in part, 2H) ; 4,25 (q, J = 7,0 Hz, 2H) ; 5,14 (s, 1H); 5,98 (d, J = 3,0 Hz, 1H) ; 6,77 (d, J = 3,0 Hz, 1 H) ; 6,82 (d large, J = 3,0 Hz, 1H) ; 6,98 (dd, J = 2,0 et 8,5 Hz, 1 H) ; 7,24 (s large, 1 H) ; 7,34 (d, J = 8,5 Hz, 1H) ; 8,30 (s, 1 H) ; 11,40 (m large, 1H)
MS : ES , m/z=517 = MH⁺.

A product in accordance with the invention may be used for the manufacture of a medicinal product that is useful for treating a pathological condition, in particular a cancer.

The present invention also relates to therapeutic compositions containing a compound according to the invention, in combination with a pharmaceutically acceptable excipient depending on the chosen mode of administration. The pharmaceutical composition may be in solid or liquid form or in the form of liposomes.

Among the solid compositions that may be mentioned are powders, gelatin capsules and tablets. Among the oral forms, solid forms protected against the acidic medium of the stomach may also be included. The supports used for the solid forms consist in particular of mineral supports such as phosphates or carbonates, or organic supports such as lactose, celluloses, starch or polymers. The liquid forms consist of solutions, suspensions or dispersions. They contain, as dispersive support, either water or an organic solvent (ethanol, NMP or the like) or mixtures of surfactants and solvents or of complexing agents and solvents.

The liquid forms will preferably be injectable and, as a result, will have a formulation that is acceptable for such a use.

Acceptable routes of administration by injection include intravenous, intraperitoneal, intramuscular and subcutaneous routes, the intravenous route being preferred.

The administered dose of the compounds of the invention will be adapted by the practitioner depending on the route of administration to the patient and the condition of said patient.

The compounds of the present invention may be administered alone or as a mixture with other anticancer agents. Among the possible combinations, that may be mentioned are:
- alkylating agents and especially cyclophosphamide, melphalan, ifosfamide, chlorambucil, busulfan, thiotepa, prednimustine, carmustine, lomustine, semustine, steptozotocin, decarbazine, temozolomide, procarbazine and hexamethylmelamine;
- platinum derivatives especially such as cisplatin, carboplatin or oxaliplatin;
- antibiotic agents especially such as bleomycin, mitomycin or dactinomycin;
- antimicrotubule agents especially such as vinblastine, vincristine, vindesine, vinorelbine or taxoids (paclitaxel and docetaxel);
- anthracyclines especially such as doxorubicin, daunorubicin, idarubicin, epirubicin, mitoxantrone or losoxantrone;
- group I and II topoisomerases such as etoposide, teniposide, amsacrine, irinotecan, topotecan and tomudex;
- fluoropyrimidines such as 5-fluorouracil, UFT or floxuridine;
- cytidine analogues such as 5-azacytidine, cytarabine, gemcitabine, 6-mercaptomurine or 6-thioguanine;
- adenosine analogs such as pentostatin, cytarabine or fludarabine phosphate;
- methotrexate and folinic acid;
- various enzymes and compounds such as L-asparaginase, hydroxyurea, trans-retinoic acid, suramin, dexrazoxane, amifostine, herceptin and estrogen and androgen hormones;
- antivascular agents such as combretastatin or colchicine derivatives and prodrugs thereof.

It is also possible to combine the compounds of the present invention with a radiation treatment. These treatments may be administered simultaneously, separately or sequentially. The treatment will be adapted to the patient to be treated by the practitioner.

A product of the invention may be useful for inhibiting the in vitro activity of an Aurora A and/or B kinase.

### Experimental protocols regarding the biochemical tests

### Aurora1 and 2 (Respectively. Aurora B and A)

The inhibitory effect of compounds with respect to the Aurora1 and 2 kinases is determined with a radioactivity scintillation assay using nickel chelate.

The kinase activity of Aurora is measured by the phosphorylation of NuMA-histidine substrate in the presence of radiolabelled ATP ([³³P] ATP) using 96 well Flash plates where the nickel-chelate is linked to the surface of the microplate. The amount of ³³P incorporated to the substrate NuMA is proportional to the aurora activity.

### Proteins:

The protein production has been made in the protein production group of Sanofi-Aventis.
- Aurora-A: the full recombinant protein including an N-terminal histidine tail has been expressed in E. coli and purified to 82%.
- Aurora-B: the Aurora-B/INCENP-C3 recombinant complex has been purified using the N-terminal poly-histidine tag to 50%.
- NuMA, (a nuclear protein which binds to the mitotic system): fragment of 424 amino acids expressed in E. coli (tagged on the extreme N-terminal with histidine for use as a the substrate for both Aurora enzymes).

### Protocol:

The Flash plates used are nickel-chelate 96 well plates (Perkin Elmer, model SMP107).

The products to be evaluated are incubated in a 100 µl reaction volume per well in the presence of 10 nM of Aurora-B or Aurora-A, 500nM of NuMA substrate in the following buffer: 50 mM of NaCl, 5 mM MgCl2, (Aurora-B) or 10 mM MgCl2 (Aurora-A) and 1 mM of DTT at 37°C.

80 µL of enzyme/substrate incubation buffer is distributed in each well, followed by 10 µL of solution of compound to be measured with various concentrations. The reaction is started by adding 1 µM of ATP (final concentration) containing 0.2 µCi of [³³P] ATP (10 µL). After 30 minutes of incubation the reaction is stopped by removal of the reaction mixture and each well is washed twice with 300 µl of buffer Tris/HCl. The radioactivity is measured in each well using a Packard Top count scintillation counter instrument.

The enzymatic activity is expressed as counts per minute obtained in 30 minutes after subtraction of the background noise (reaction medium without enzyme). The measurement is expressed as percentage of inhibition of Aurora activity versus the control.

**Table 16: inhibitory activity of example compounds towards Aurora A kinase.**

| Examples | IC50 uM | | Examples | IC50 uM |
|---|---|---|---|---|
| 3 | 0.2 | | | |
| 4 | 0.022 | | 60 | 6.5 |
| | | | 61 | 11.4 |
| | | | 62 | 37 |
| | | | 63 | 3.3 |
| | | | 64 | 4.1 |
| | | | 65 | 0.63 |
| | | | 66 | 11 |
| | | | 67 | 2 |
| | | | 68 | 36 |
| | | | 69 | 20 |
| | | | 70 | 0.03 |
| | | | 71 | 0.16 |
| | | | 72 | 0.019 |
| | | | 73 | 0.029 |
| | | | 74 | 0.17 |
| | | | 75 | 0.062 |
| 20 | 0.1 | | 76 | 0.034 |
| | | | 77 | 1.2 |
| | | | 78 | 0.2 |
| | | | 79 | 0.062 |
| | | | 80 | 0.43 |
| | | | 81 | 0.029 |
| | | | 82 | 0.11 |
| | | | 83 | >10 |
| 28 | 0.067 | | 84 | 0.82 |
| 29 | 0.1 | | 85 | 0.02 |
| 30 | 0.05 | | 86 | 0.065 |
| 31 | 0.067 | | 87 | 0.015 |
| 32 | 0.13 | | 88 | 0.36 |
| 33 | 0.18 | | 89 | 0.17 |
| 34 | 0.053 | | 90 | 0.75 |
| 35 | 0.04 | | 91 | 0.14 |
| 36 | 0.1 | | 92 | |
| 37 | 0.19 | | 93 | |
| 38 | 0.097 | | 94 | |
| 39 | 0.13 | | 95 | |
| 40 | 11.1 | | 96 | |
| 41 | 0.026 | | 97 | |
| 42 | 0.47 | | 98 | |
| 43 | 10 | | 99 | |
| 45 | 1.5 | | 100 | |
| | | | 101 | |
| | | | 102 | |
| | | | 103 | |
| | | | 104 | 0.12 |
| | | | 105 | 0.01 |
| | | | 106 | 0.008 |
| | | | 107 | 3.45 |

## Claims

1. Compound corresponding to the general formula (I) below 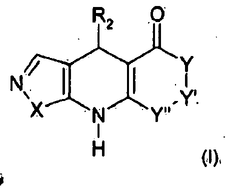 wherein:
X is NH or CR₇ and
• when X=NH, R₂ is:
- aryl substituted with a substituent selected among S-R₉, NH-R₉, (C=O)-NH-R₉, (C=O)-NH-CH₂-R₉, NH-(C=O)-R₉, NH-(C=O)-NH-R₉, (SO₂)-NH-R₉
or
- heteroaryl substituted with a substituent selected among O-R₉, S-R₉, NH-R₉, (C=O)-NH-R₉, (C=O)-NH-CH₂-R₉, NH-(C=O)-R₉, NH-(C=O)-NH-R₉, (SO₂)-NH-R₉
• when X= CR₇, R₂ is aryl or Heteroaryl substituted with a substituent selected among O-R₉, S-R₉, NH-R₉, (C=O)-NH=R₉, (C=O)-NH=CH₂-R₉, NH-(C=O)-R₉, NH-(C=O)=NH-R₉, (SO₂)-NH-R_{9;}
• R₉ is chosen among aryl and heteroaryl optionally substituted with a substituent chosen among H, F, Cl,. Br, OH, SH, CF_{3,} OCF₃, OCH₃, SCF_{3,} SCH₃, OCHF₂, OCH₂F, (C1-C6)alkyl, O-allyl, phenyl, and phenyl substituted with halogen ;
• Y, Y' and Y" :
(i) each independently represent a substituent selected among CH₂, CHR₅, CR₅R₆. C=O, O, S, NH, and NR₇; or
(ii) together represent a substituent selected among -CH₂-O-(C=O)-, -(CH₂)₄- and -(CH₂)₂-chain moiety;
• R₇ represent a substituent selected among: R₈, COOR₈, COR₈, and tONHR₈ ;
• R₅ and R₆ each independently represent R₈ ;
• R₈ represents H or optionally substituted: -alkyl, -alkyl-alkylene, -alkylene, -heterocycloalkyl, - cycloalkyl, -aryl, -heteroaryl, -alkyl-heterocycloalkyl, -alkyl-cycloalkyl, -alkyl-aryl, or -alkylheteroaryl.

2. Compound according to claim 1 wherein Y" is CH₂.

3. Compound according to claim 1 or 2 wherein Y is CH₂.

4. Compound according to claim 1 to 3 wherein Y' is selected among CH₂, CHCH₃, C(CH₃)₂, CH-aryl, CH-heteroaryl, CH-(substituted aryl), CH-(substituted heteroaryl), O, S, NH, and NR₇.

5. Compound according to any one of claims 1 to 4 wherein R₂ is a heteroaryl substituted by SR₉:

6. Compound according to claim 5 wherein R₉ is a optionally substituted heteroaryl,

7. Compound according to claims 1 to 6 wherein R₉ is an optionally substituted benzimidazolyl or an optionally substituted Imidazolyl.

8. Compound according to claim 1 wherein R₂ is substituted heteroaryl wherein heteroaryl is furyl or thienyl.

9. Compound according to any one of claims 1 to 7 wherein X is CR₇.

10. Compound according to claim 8 wherein R₇ is COOR₈ or CONHR₈.

11. Compound according to any one of the preceding claims, in the racemic form, enriched in one enantiomer, enriched in one diastereoisomer, its tautomers, its prodrugs and its pharmaceutically acceptable salts.

12. Compound according to any one of claims 1 to 11 as a drug.

13. Compound according to any one of claims 1 to 11 as an anticancer drug.

14. Use of a product according to any one of claims 1 to 12 for producing a medicinal product of use in treating a pathological condition.

15. Use according to claim 14 wherein the pathological condition is chosen among cancer, psoriasis, leukaemia and lupus.

16. Use according to claim 14 wherein the pathological condition is cancer.

## Patentansprüche

1. Verbindung der nachstehenden allgemeinen Formel (I) : 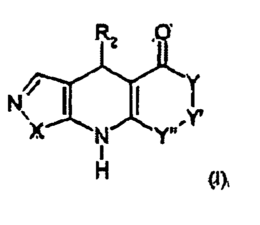 worin:
X für NH oder CR₇ steht und
• dann, wenn X = NH, R₂ für:
- Aryl, das durch einen unter S-R₉, NH-R₉, (C=O) -NH-R₉, (C=O) -NH-CH₂-R₉, NH- (C=O) -R₉, NH-(C=O) -NH-R₉ und (SO₂) -NH-R₉ ausgewählten Substituenten substituiert ist,
oder
- Heteroaryl, das durch einen unter O-R₉, S-R₉, NH-R₉, (C=O) -NH-R₉, (C=O) -NH-CH₂-R₉, NH- (C=O) - R₉, NH- (C=O) -NH-R₉ und (SO₂)-NH-R₉ ausgewählten Substituenten substituiert ist,
steht;
• dann, wenn X = CR₇, R₂ für Aryl oder Heteroaryl, das durch einen unter O-R₉, S-R₉, NH-R₉, (C=O) - NH-R₉, (C=O) -NH-CH₂-R₉, NH- (C=O) -R₉, NH- (C=O) -NH-R₉ und (SO₂)-NH-R₉ ausgewählten Substituenten substituiert ist, steht;
• R₉ unter Aryl und Heteroaryl, gegebenenfalls substituiert durch einen unter H, F, C1, Br, OH, SH, CF₃, OCF₃ , OCH₃ , SCF₃ , SCH₃ , OCHF₂ , OCH₂F, (C1-C6)-Alkyl, O-Allyl, Phenyl und halogensubstituiertem Phenyl ausgewählten Substituenten, ausgewählt ist;
• Y, Y' und Y" :
(i) jeweils unabhängig voneinander für einen unter CH₂, CHR₅, CR₅R₆, C=O, O, S, NH und NR₇ ausgewählten Substituenten stehen oder
(ii) gemeinsam für einen unter -CH₂-O-(C=O)-,
-(CH₂)₄- und - (CH₂)₂-Ketteneinheiten ausgewählten Substituenten stehen;
• R₇ für einen unter R₈, COOR₈, COR₈ und CONHR₈ ausgewählten Substituenten steht;
• R₅ und R₆ jeweils unabhängig voneinander für R₈ stehen;
• R₈ für H oder gegebenenfalls substituiertes -Alkyl, -Alkylalkylen, -Alkylen, -Heterocycloalkyl, -Cycloalkyl, -Aryl, -Heteroaryl, -Alkyl-heterocycloalkyl, -Alkylcycloalkyl, -Alkylaryl oder -Alkylheteroaryl steht.

2. Verbindung nach Anspruch 1, worin Y " für CH₂ steht.

3. Verbindung nach Anspruch 1 oder 2, worin Y für CH₂ steht.

4. Verbindung nach Anspruch 1 bis 3, worin Y' unter CH₂, CHCH₃, C(CH₃)₂, CH-Aryl, CH-Heteroaryl, CH-(substituiertes Aryl), CH-(substituiertes Heteroaryl), O, S, NH und NR₇ ausgewählt ist.

5. Verbindung nach einem der Ansprüche 1 bis 4, worin R₂ für ein durch SR₉ substituiertes Heteroaryl steht.

6. Verbindung nach Anspruch 5, worin R₉ für gegebenenfalls substituiertes Heteroaryl steht.

7. Verbindung nach einem der Ansprüche 1 bis 6, worin R₉ für ein gegebenenfalls substituiertes Benzimidazolyl oder ein gegebenenfalls substituiertes Imidazolyl steht.

8. Verbindung nach Anspruch 1, worin R₂ für substituiertes Heteroaryl, worin Heteroaryl Furyl oder Thienyl bedeutet, steht.

9. Verbindung nach einem der Ansprüche 1 bis 7, worin X für CR₇ steht .

10. Verbindung nach Anspruch 8, worin R₇ für COOR₈ oder CONHR₈ steht.

11. Verbindung nach einem der vorhergehenden Ansprüche in racemischer Form, mit einem Enantiomer angereichert, mit einem Diastereoisomer angereichert, Tautomere davon, Prodrugs davon und pharmazeutisch akzeptable Salze davon.

12. Verbindung nach einem der Ansprüche 1 bis 11 als Arzneistoff.

13. Verbindung nach einem der Ansprüche 1 bis 11 als Antikrebs-Arzneistoff.

14. Verwendung eines Produkts nach einem der Ansprüche 1 bis 12 zur Herstellung eines medizinischen Produkts zur Verwendung bei der Behandlung eines pathologischen Zustands.

15. Verwendung nach Anspruch 14, bei der der pathologische Zustand unter Krebs, Psoriasis, Leukämie und Lupus ausgewählt ist.

16. Verwendung nach Anspruch 14, bei der es sich bei dem pathologischen Zustand um Krebs handelt.

## Revendications

1. Composé correspondant à la formule générale (I) ci-dessous : 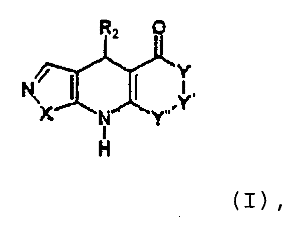 dans laquelle : X est NH ou CR₇ et
• lorsque X = NH, R₂ est
- un aryle substitué par un substituant choisi parmi S-R₉, NH-R₉ (C=O) -NH-R₉ (C=O)-NH-CH₂-R₉, NH- (C=O) - R₉, NH- (C=O) -NH-R₉, (SO₂) -NH-R₉
ou
- un hétéroaryle substitué par un substituant choisi parmi O-R₉, S-R₉, NH-R₉, (C=O) -NH-R₉ (C=O) -NH-CH₂-R₉, NH- (C=O) -R₉ NH- (C=O) -NH-R₉ (SO₂) -NH-R₉ ;
• lorsque X = CR₇, R₂ est un aryle ou hétéroaryle substitué par un substituant choisi parmi O-R₉ S-R₉, NH-R₉ (C=O) -NH-R₉ (C=O) -NH-CH₂-R₉, NH- (C=O) -R₉, NH-(C=O) -NH-R₉ (SO₂) -NH-R₉ ;
• R₉ est choisi parmi un aryle et hétéroaryle facultativement substitué par un substituant choisi parmi H, F, Cl, Br, OH, SH, CF₃, OCF₃, OCH₃, SCF₃, SCH₃, OCHF₂, OCH₂F, alkyle en C1-C6, O-allyle, phényle et phényle substitué par un halogène ;
• Y, Y' et Y" :
(i) représentent chacun indépendamment un substituant choisi parmi CH₂, CHR₅, CR₅R₆, C=O, O, S, NH et NR₇ ; ou
(ii) représentent ensemble un substituant choisi parmi une fraction de chaîne -CH₂-O-(C=O)-,
-(CH₂)₄- et -(CH₂)₂- ;
• R₇ représente un substituant choisi parmi : R₈, COOR₈, COR₈ et CONHR₈ ;
• R₅ et R₆ représentent chacun indépendamment R₈ ;
• R₈ représente H ou un -alkyle, -alkylalkylène, -alkylène, -hétérocycloalkyle, -cycloalkyle, -aryle, -hétéroaryle, -alkylhétérocycloalkyle, -alkylcycloalkyle, - alkylaryle ou -alkylhétéroaryle facultativement substitué.

2. Composé selon la revendication 1 dans lequel Y" est CH₂.

3. Composé selon la revendication 1 ou 2 dans lequel Y est CH₂.

4. Composé selon la revendication 1 à 3 dans lequel Y' est choisi parmi CH₂, CHCH₃, C(CH₃)₂, CH-aryle, CH-hétéroaryle, CH-(aryle substitué), CH-(hétéroaryle substitué), O, S, NH et NR₇.

5. Composé selon l'une quelconque des revendications 1 à 4 dans lequel R₂ est un hétéroaryle substitué par SR₉.

6. Composé selon la revendication 5 dans lequel R₉ est un hétéroaryle facultativement substitué.

7. Composé selon les revendications 1 à 6 dans lequel R₉ est un benzimidazolyle facultativement substitué ou un imidazolyle facultativement substitué.

8. Composé selon la revendication 1 dans lequel R₂ est un hétéroaryle substitué, lequel hétéroaryle est un furyle ou thiényle.

9. Composé selon l'une quelconque des revendications 1 à 7 dans lequel X est CR₇.

10. Composé selon la revendication 8 dans lequel R₇ est COOR₈ ou CONHR₈.

11. Composé selon l'une quelconque des revendications précédentes sous la forme racémique, enrichi en un énantiomère, enrichi en un diastéréoisomère, tautomères de celui-ci, promédicaments de celui-ci et sels pharmaceutiquement acceptables de celui-ci.

12. Composé selon l'une quelconque des revendications 1 à 11 comme médicament.

13. Composé selon l'une quelconque des revendications 1 à 11 comme médicament anticancéreux.

14. Utilisation d'un produit selon l'une quelconque des revendications 1 à 12 pour produire un produit médicinal utile dans le traitement d'un état pathologique.

15. Utilisation selon la revendication 14 dans laquelle l'état pathologique est choisi parmi le cancer, le psoriasis, la leucémie et le lupus.

16. Utilisation selon la revendication 14 dans laquelle l'état pathologique est le cancer.
